# EUROPEAN PATENT APPLICATION

(11) **EP 2 821 082 A1**
(43) Date of publication of application: **07.01.2015**
(21) Application number: 13305966.7
(22) Date of filing: 05.07.2013
(51) Int. Cl.: A61K 39/12

(54) **Method of producing an inactivated lentivirus, especially HIV, vaccine, kit and method of use**

(71) Applicant: LABORATOIRE BIODIM, 75007 Paris (FR)
(72) Inventor: Benarous, Richard, 75013 Paris (FR); Le Rouzic, Erwann, 75011 Paris (FR); Bruneau, Jean-Michel, 75020 Paris (FR); Bonnard, Damien, 75019 Paris (FR); Moreau, François, 91400 Orsay (FR)
(74) Representative: Colombet, Alain André

(57) **Abstract**

A novel method for producing an immunogenic composition or vaccine comprising inactivated lentivirus, in particular inactivated HIV, wherein producer cells constituvely producing lentivirus particles are provided, the lentivirus particles are produced by these producer cells in the presence of an antiretroviral (ARV) agent which is an inhibitor of the IN-LEDGF/p75 interaction, the inactivated lentivirus is recovered and formulated in a pharmaceutically acceptable vehicle or carrier. The invention also relates to immunogenic compositions or vaccines and to methods for the therapeutic or prophylactic treatment of a mammal, especially a human, and various therapy combinations involving the administration of said immunogenic compositions or vaccines.

## Description

### Introduction

The Acquired Immuno Deficiency Syndrome (AIDS) is a disease due to infection by the Human Immunodeficiency Virus (HIV). HIV is a retrovirus, belonging to the subclass of primate lentiviruses. Two types of HIV have been identified, HIV-1 and HIV-2. HIV-1 is responsible for the larger part of the AIDS global epidemic in the world, with virtually every country reporting cases.

The AIDS pandemic is a major global public-health threat with an estimated 34 million people infected with HIV, mainly in Sub-Saharan Africa. Highly active antiretroviral therapy (HAART), i.e. combination therapy of three or more antiretroviral drugs with different mechanisms of action, is very effective at controlling HIV replication and has reduced both HIV-associated mortality and morbidity. However, none of these ARV drugs nor any HAART regimen are able to eradicate and cure HIV that is maintained at very low copy number, integrated but dormant, non expressed in various cell reservoirs. Thus patients need to take their HAART treatment for life with strict compliance. In addition, with patients failing current ARV treatments and with expansion of ARV therapy programs, the emergence and transmission of drug-resistant viruses has become a new serious public health.

Several modalities can reduce HIV-1 infection rates in persons at risk of exposure, such as screening of blood banks and blood-derived products, counseling campaigns for the use of condoms, pre- or immediate post-exposure antiretroviral therapy (ART), male circumcision, use of ARV agents as microbicides in vaginal gel.

A highly efficacious vaccine that would prevent HIV infection and/or could cure HIV infected patients is therefore urgently needed. Unfortunately, despite more than 20 years of intense search no effective vaccine is currently available nor envisioned in the near future. Numerous candidate HIV or SIV vaccines have been developed which elicited varying degrees of protective responses in nonhuman animal models, including DNA vaccines, subunit vaccines, live attenuated or inactivated recombinant vaccines, various prime-boost combinations, eliciting non neutralizing or broadly neutralizing antibodies capable of inhibiting circulating viruses, and protection by passive immunization. (For review see Saunders et al. AIDS. 2012 Jun 19;26(10):1293-302, or Marc P. Girard et al. Vaccine, Vol 29, Issue 37, 26 August 2011, Pages 6191-6218). Four of these candidate vaccines have been tested for efficacy in human volunteers, but, to the exception of the recent RV144 Phase III trial in Thailand, which elicited a modest level of partial protection against infection (S. Rerks-Ngarm et al., N Engl J Med, 361 (2009), pp. 2209-2220; D.R. Burton et a/., Science, 303 (2004), p. 316), none has shown efficacy in preventing HIV infection or in controlling virus replication and delaying progression of disease in humans. Thus new strategies are needed to overcome the obstacles to the development of vaccines capable to efficiently prevent HIV infection and/or control HIV replication and delay disease progression in infected individuals, alone or in combination with another therapy.

This is the purpose of the present work to propose such innovative approach and protocols toward efficient anti-HIV vaccines.

From the numerous studies performed to date, it is believed that to develop efficacious anti-HIV vaccine, potent immunogens are required to generate both cell-mediated and antibody-mediated responses. Among the strategies used toward this goal, several attempts have been performed using HIV or SIV Gag and Env proteins assembled in virus-like particles (VLP) that contained only the viral core and Env proteins, or HIV viruses inactivated after their production and isolation as cell-free virus, by treatment with several denaturing and inactivating agents such as zinc chelators (2,2'-dithiobisbenzamide (DIBA)), heat, UV or cross-linking agents such as psoralen, or a combination of several of these methods (J.D. Lifson et a/., AIDS Res Hum Retroviruses, 20 (2004), pp. 772-787; L.X. Doan et al., Rev Med Virol, 15 (2005), pp. 75-88; Gil C et al., Vaccine. 2011 Aug 5;29(34):5711-24. None of these methods have resulted in efficient vaccine candidate.

Recently developed ARV compounds acts by a dual mechanism of: i) inhibition of HIV replication in target cells by binding to HIV-1 Integrase (IN) and inhibition of IN-LEDGF interaction, and ii) inactivation of HIV viruses released by producer cells upon compound binding to the LEDGF-binding pocket of IN resulting in inactivation of IN through enhancement of IN-IN subunits interaction: Christ, F. et a/. (2012) Antimicrob. Agents Chemother. 56, 4365-4374 ; E. Le Rouzic et al.; abstract #547, CROI conference March 3-6, 2013, Atlanta, USA; Stephen Yant et al., abstract #103 at the CROI conference March 3-6, 2013, Atlanta, USA,; B. Desimmie et al; Abstract #104 CROI conference March 3-6 2013, Atlanta, USA, B. Desimmie et al. Abstract #138, CROI conference March 3-6, 2013, Atlanta, USA; Kellie A. Jurado et al. Proc. Natl. Acad. USA, 2013 May 21;110(21):8690-5. Desimmie BA. et al, Retrovirology 2013 May 30;10:57. doi: 10.1186/1742-4690-10-57. Mention of some references is not a recognition that the reference is or relates to prior art or is relevant to patentability with respect to the present application.

LEDGF/p75 is a cofactor of IN that binds to IN through the IN-catalytic core domain (IN-CCD) and this interaction is required for the integration of the HIV proviral DNA to actively transcribed genes of the host genome. The integrase binding domain (IBD) on LEDGF/p75 is located toward the C-terminus of the protein and is absent in the LEDGF/p52 isoform (for review see Engelman, A., and Cherepanov, P. (2008), PLoS Pathog 4, e1000046).

### Summary of the invention

Within the invention it has been found that, by contrast with HIV denaturing agents such as zinc chelators, heat, UV or cross-linking agents, these ARV compounds do not inactivate HIV after virus production and isolation as cell-free virus, but inactivate HIV only during virus production intracellularly, upon treatment of producer cells. The drawback with denaturing agents that are used to treat cell-free viruses after their release from producer cells is that they strongly denature the overall structure of the HIV virus particles as well as particular components of these particles, thus altering their immunogenicity. Without willing to be bound to theory, it is deemed the advantages of the use of the compounds that inactivate HIV during their production intracellularly in producer cells are the followings:
1- the inactivated virus particles are not denatured, are apparently normally matured with normal Capsid content, fully matured precursor Gag protein, and are released similarly with normal untreated HIV, with the viral envelope and normal p24 reactive virus particles in the supernatant of producer cells. However these HIV virus particles inactivated during their production, when used to infect various cells target of HIV infection (target cells), are unable to infect and replicate in these target cells and thus are defective, preferably fully defective for HIV infection. The reason of such inactivation is related to an irreversible conformational modification of HIV integrase promoted by compound binding to the LEDGF-binding pocket on HIV integrase.
2- The HIV viruses inactivated in accordance with the invention have several peculiarities, namely an abnormal multimerization of their integrase that can be detected, e.g. by cross linking experiment or by Fluorescence energy transfer (FRET).
3- Such HIV virus particles inactivated during their production in producer cells should be much better immunogens than virus particles inactivated after production, since they are not denatured virus particles. Interestingly, in contrast with denatured HIV particles, these inactivated viruses can enter normally in target cells of HIV infection (Stephen Yant et al. ; Abstract #104 CROI conference March 3-6 2013, Atlanta, USA), but their replication is blocked at the reverse transcription stage which is completely impaired. By entering target cells inactivated viruses should induce a CD8+ cellular immunity response that cannot be promoted by denatured viruses.
4- These compounds that inactivate HIV virus particles upon treatment of producer cells during the intracellular process of virus production, are true antiretroviral compounds that can be used to treat HIV infected patients, and could be combined with anti-HIV vaccine, which is not the case of the denaturing agents previously used to inactivate HIV after their release as cell-free virus, since these denaturing agents are not specific to HIV and are poisonous highly toxic agents that cannot be administered to humans or animals.
5- The inactivation of HIV upon treatment of producer cells according to the invention requires the binding of the ARV compound to the LEDGF-binding pocket on HIV integrase, as exemplified by co-crystallization of these compounds with the HIV-1 integrase Catalytic Core Domain of (IN-CCD) (see e.g. example 1).
6- On the one hand, these ARV compounds inactivate HIV by acting on producer cells during virus production, similarly as Protease Inhibitor (PI) drugs act. However, on the other hand, while PIs inactivate HIV by inhibiting precursor Gag maturation with altered profile of Gag maturation and low or absence of Capsid content of virus particles, compounds subject of the invention inactivate HIV without any apparent alteration of Gag maturation or Capsid content of the inactivated viruses.

Interestingly, all lentiviruses are dependent on interaction between their integrase and LEDGF from the host in order to be properly integrated in the host genome (Cherepanov P. Nucleic Acids Res. 2007;35(1):113-24; Llano M et al., J Virol. 2004 Sep;78(17):9524-37; Kang Y et al., J Virol. 2006 Sep;80(17):8820-3.; Busschots K et al., J Biol Chem. 2005 May 6;280(18):17841-7). Thus, the protocol of virus inactivation using compounds according to the invention, in particular that bind to the LEDGF binding site and multimerize integrase, can also be applied to the inactivation of these other lentiviruses and exploited for vaccine design for human or veterinary use.

There are currently 26 licensed drugs falling into 7 different classes: 7 nucleoside reverse transcriptase inhibitors (NRTIs), 1 nucleotide reverse transcriptase inhibitor (NtRTI), 4 non-nucleoside reverse transcriptase inhibitors (NNRTIs), 10 protease inhibitors (PIs), 1 fusion inhibitor (FI), 1 co-receptor inhibitor (CRI) and 2 integrase strand transfer inhibitor (INSTI) (De Clercq, Erik, Antiretroviral drugs. Curr Opin Pharmacol, 2010. 10(5): p. 507-515). Protease inhibitors are the only class of licensed ARV drugs that act at the stage of virus production in producer cells. All the other classes of drugs act at various steps early in the replication cycle of HIV to block infection of target cells, and are inactive at the level of HIV production in producer cells. ARV compounds subjects of the invention are a new class of compounds that have a unique dual mechanism of action, inactivation of HIV at the level of HIV production in producer cells, and also inhibition of HIV replication at the level of target cells.

The present invention relates to the use of the ability of these compounds to inactivate lentiviruses, especially HIV, upon production in producer cells in order to produce inactivated virus that will be used as a new type of immunogen in an antilentivirus, especially anti-HIV, vaccine or immunogenic composition.

A first object of the invention is thus a method for producing an immunogenic composition or vaccine comprising inactivated lentivirus, in particular inactivated HIV, wherein producer cells constituvely producing lentivirus particles are provided, the lentivirus particles are produced by these producer cells in the presence of an antiretroviral (ARV) agent which is an inhibitor of the IN-LEDGF/p75 interaction, the inactivated lentivirus is recovered and formulated in a pharmaceutically acceptable vehicle or carrier.

A second object of the invention is an immunogenic composition or vaccine comprising an inactivated lentivirus, especially inactivated HIV, in a pharmaceutically acceptable carrier or vehicle, and optionally an adjuvant, obtained using the method as disclosed herein.

A third object of the invention is an immunogenic composition or vaccine comprising an inactivated lentivirus, especially inactivated HIV, in a pharmaceutically acceptable carrier or vehicle, and optionally an adjuvant, wherein the lentivirus comprises a tetramer of integrase.

A fourth object of the invention is an immunogenic composition or vaccine comprising an inactivated lentivirus, especially inactivated HIV, in a pharmaceutically acceptable carrier or vehicle, and optionally an adjuvant, wherein the lentivirus comprises an inactive integrase having a MW of about 130 KD corresponding to an integrase tetramer as measured using the method of chromatography on a Superdex PC 3.2/30 column (GE Healthcare)

A fifth object of the invention is a kit for prophylactic or therapeutic treatment of a mammal, especially a human, against a lentivirus, especially HIV, comprising an immunogenic composition or vaccine according to claim 21, 22 or 23 and a antiretroviral (ARV) agent which is an inhibitor of the IN-LEDGF/p75 interaction, for a simultaneous, separate or sequential administration.

A sixth object of the invention is a method of prophylactic or therapeutic treatment of a mammal, especially a human, against a lentivirus, especially HIV, comprising administering an effective amount of an immunogenic composition or vaccine according to the invention.

### Definitions:

- Producer cells: Cells capable of producing lentivirus particles, especially HIV particles, in particular of subtype HIV-1 or HIV-2, released in the extracellular medium, upon previous HIV infection, or after suitable DNA transfection, e.g. using a plasmid harbouring a full length HIV proviral DNA construct. Producer cells could be any cells infected by HIV and harbouring CD4 receptor and co-receptor either CCR5 or CXCR4 that are required for HIV-infection, such as Peripheral Blood Mononuclear Cells (PBMC), or macrophages, isolated from HIV-infected or non infected person, transformed CD4+ lymphocytic cell lines such as MT4, MT2, Jurkat cell lines, any cells that could be transfected with plasmid harbouring HIV infectious clones, such as 293T cells, Hela cells.
- Target cells: Cells harbouring or not a reporter gene that can be infected by HIV and that harbour at their surface receptor (CD4) and co-receptor (CCR5 or CXCR4 for HIV R5 and X4 tropism respectively) that are needed for infection by HIV.
- Antiretroviral (ARV) agent: a small chemical product or molecule including peptides that is capable to inhibit HIV replication from infected cells, and that can be developed and used as anti-HIV drug to treat HIV infected patients or animal models of HIV infection.
- Inactivate HIV: any treatment capable to suppress the ability of HIV to replicate, *in vitro* in infected cells and *in vivo* in animal models of HIV infection, and to be transmitted and spread in surrounding non infected cells.
- HIV Inactivating agent: ARV agent capable to inactivate HIV, and thus to suppress its ability to replicate in producer cells and infect target cells *in vitro* and replicate *in vivo* in infected patients or animal models of HIV infection.
- HIV denaturating agent: agent capable to inactivate HIV after its production and release as cell-free virus in the extracellular medium.
- Vaccine preparation with ARV-inactivated HIV: use of a preparation, preferably a purified preparation, of inactivated HIV obtained after treatment of HIV producer cells with ARV-inactivating agent.
- Prophylactic HIV vaccine: vaccine preparation used to prevent HIV infection in uninfected individuals or animal models of HIV infection.
- Therapeutic HIV vaccine: vaccine preparation used in combination with ARV therapy in order to boost the ARV treatment efficiency, either by enhancement of immune reconstitution, increase of CD4 count and/or maintaining very low viral load after ARV treatment interruption ultimately toward cure of HIV infection.
- Autologous HIV molecular clones: HIV molecular clones isolated and prepared from a particular HIV-infected individual that will be inactivated and used to vaccine the same individual from whom they were isolated for a therapeutic vaccine purpose.
- Heterologous HIV molecular clones: HIV isolated and cloned from previously available source (databank) different from HIV clones prepared from an HIV-infected individual.
- Immunogenic composition covers any composition capable, once administered to the target species, in particular a human subject, under the conditions of the invention, of inducing an immune response directed against the lentivirus. The immune response may not be protective or sufficiently protective alone, for prophylaxis and/or therapy.
- Vaccine is intended to mean a composition capable of inducing effective prophylactic and/or therapeutic protection or able to contribute to such a protection with another vaccine composition or ingredient and/or an antiviral therapy such as with an ARV compound according to the invention.
- Adjuvant: substance that acts as agent to enhance the effectiveness of the vaccine composition. Examples of vaccine adjuvants can be found in Recent advances in vaccine adjuvants, Singh M. & O'Hagan D.T. Pharmaceutical research 2002, Volume 19, Issue 6, pp 715-728; The use of conventional immunologic adjuvants in DNA vaccine preparations, Sasaki S. & Okuda K., in DNA Vaccines Methods in Molecular Medicine™, Volume 29, 2000, pp 241-249; Sayers, S et al. Journal of biomedicine & biotechnology 2012: 831486, PMID 22505817. An immunologic adjuvant is defined as any substance that acts to accelerate, prolong, or enhance antigen-specific immune responses when used in combination with specific vaccine antigens. Immunologic adjuvants include certain cytokines and other immunomodulatory molecules (i.e. chemokines and costimulatory factors) or their expression vectors, adjuvants that are derived from microorganisms and plants or are synthesized chemically. Adjuvants can act as: vaccine delivery systems and immunostimulatory adjuvants. Vaccine-delivery systems generally are particulate (e.g., emulsions, microparticles, ISCOMS, and liposomes) and function mainly to target associated antigens into antigen-presenting cells. In contrast, immunostimulatory adjuvants are derived predominantly from pathogens and often represent pathogen-associated molecular patterns (e.g., lipopolysaccaride, monophosphoryl lipid A, CpG DNA), which activate cells of the innate immune system. Mucosal adjuvants may also allow vaccines to be delivered mucosally. Mucosal adjuvants suitable for use in the invention include but are not limited to E. coli heat labile enterotoxins, detoxified mutants such as K63 or R72, adjuvants that favor immune tolerance, such as *Lactobacillus plantarum* (Kleerebezem et al. Proc. Natl. Acad. Sci. USA, 100, 1990-1995, 2003, Van Baarlen et al. Proc. Natl. Acad. Sci. USA, 106, 2371-2376, 2009).

Among the adjuvants which may be used, there may be mentionned by way of example, aluminium hydroxide, the saponines (e.g. Quillaja saponin or Quil A; see Vaccine Design, The Subunit and Adjuvant Approach, 1995, edited by Michael F. Powel and Mark J. Newman, Plennum Press, NY and London, p. 210), Avridine® (Vaccine Design p.148), DDA (dimethyldioactadecyl-ammonium bromide, Vaccine Design p.157), polyphosphazene (Vaccine Design p.204), oil-in-water emulsions, in particular based on mineral oil, squalene (e.g. SPT emulsion, Vaccine Design p. 147), squalene (e.g. MF59, Vaccine Design p.183), water-in-oil emulsions, particularly based on metabolizable oil (such as according to WO9420071), an emulsion according to US 5,422,109, triple emulsions such as water-in-oil-in-water emulsions.
- Pharmaceutically acceptable refers to a carrier or vehicle or excipient that can be used to administrate the immunogen to the subject without allergic or other adverse effect. This includes, for example, one or more of water, saline, phosphate buffered saline, dextrose, glycerol, ethanol, or the like and combinations thereof. By way of example, it may be a 0.9% NaCl saline solution or a phosphate buffer. The composition may vary depending on the route of administration in accordance with knowledge in the art.

### Detailed description of the invention:

The present invention thus relates first to a method for producing an immunogenic composition or vaccine comprising inactivated lentivirus, in particular inactivated HIV, wherein producer cells constituvely producing lentivirus particles are provided, the lentivirus particles are produced by these producer cells in the presence of an antiretroviral (ARV) agent which is an inhibitor of the IN-LEDGF/p75 interaction, the inactivated lentivirus is recovered and formulated in a pharmaceutically acceptable vehicle or carrier.

In addition to human lentiviruses, especially HIV, lentiviruses concerned by the invention include, but are not limited, to SIV, SHIV, FIV, CAEV, EIAV, BIV.

According to a feature, the inactivated lentivirus is recovered and formulated in a pharmaceutically acceptable vehicle or carrier and an adjuvant.

According to a feature, the inactivated lentivirus is recovered and formulated in a pharmaceutically acceptable vehicle or carrier, optionally an adjuvant, and the formulation is sterilized.

According to a feature, the producer cell is a cell line which expresses constituvely lentivirus particles.

According to a feature, the producer cells are transfected with a plasmid harboring full length lentiviral proviral DNA construct.

According to a feature, the producer cell harbour CD4 receptor and/or the co-receptor CCR5 and/or CXCR4.

According to a feature, the inactivated lentivirus comprises a multimerized form of inactive integrase having a molecular weight greater than the integrase dimer.

According to a feature, the inactivated lentivirus comprises an inactive tetramer of integrase.

According to a feature, the inactivated lentivirus comprises an inactivated integrase multimer resulting from a shift toward higher order oligomerisation, preferably an inactived integrase tetramer of about 130 KD MW, that can be detected by cross linking experiment, by Fluorescence energy transfer (FRET) or using the method of size exclusion chromatography preferably on a Superdex PC 3.2/30 column (GE Healthcare).

The present method first comprises providing a producer cell which is capable of constituvely producing the lentivirus. In an embodiment the method first comprises providing a producer cell which is capable of constituvely producing HIV, especially HIV-1 or HIV-2.

According to an embodiment, providing a producer cell comprises the production of the producer cell. Production of a producer cell comprises transfecting a suitable cell with a construction comprising lentivirus, for example HIV, proviral DNA.

According to a feature, the method of production comprises the preparation of a plasmid or cloning vector and the like harboring an infectious lentivirus molecular clone. The lentivirus may be HIV. The molecular clone may be a previously cloned virus issued from a biobank or isolated from a lentivirus infected individual (autologous HIV). The molecular clone may also be prepared from the quasi species population of lentivirus that infects a patient. Molecular cloning of lentiviruses, especially HIV, is known to the person skilled in the art. As a general reference, see Russell David W. and Sambrook Joseph, 2001, Molecular Cloning: a laboratory manual. Cold Spring Harbor, N.Y.: Cold Spring Harbor Laboratory.

One may obtain infectious HIV molecular clones from various subtype origin, HIV-1 or HIV-2. These molecular clones could be previously cloned HIV such as pNL4-3 (Adachi A et al. J Virol 59:284-291, 1986), pYU2 (Li Y et al. J Virol 65:3973-3985, 1991), p89.6 pLAI (Collman R et al. J Virol 66:7517-7521, 1992.) or any infectious HIV molecular clone previously described and available in various databank, in particular with X4 or R5 tropism, from various subtypes, such as but not limited to those listed in the NIH AIDS Reagent Program (https://www.aidsreagent.org/Index.cfm).

Alternatively and importantly several infectious HIV viruses representative of the quasi species population of HIVs that infect a particular patient, treated or untreated by ARV therapy, can be cloned from the patient, giving rise to autologous HIV molecular clones. These autologous infectious HIV molecular clones can be prepared using HIV released from Peripheral Blood Mononuclear Cells (PBMC) from infected patients, or from plasma viral RNA isolation, then PCR amplification, construction of HIV full-length infectious molecular clones. Full-length HIV fragments generated by PCR amplification may be purified and cloned in bacterial plasmids by an appropriate method, such as using the TOPO XL PCR Cloning Kit (Invitrogen). These plasmids may be cultured at a suitable temperature, e.g. 30°C. The plasmids may be purified using for example the QIAprp Miniprep kit (Qiagen). Plasmids harboring HIV clones may be checked for insert size and sequence, and expanded. These clones may be constructed according the methods described by Ehrenberg PK & Michael NL PCR amplification, cloning, and construction of HIV-1 infectious molecular clones from virtually full-length HIV-1 genomes in Human retrovirus Protocols, Methods in Molecular Biology vol. 304, 2005, pp387-398), or by Rousseau CM et al. Large-scale amplification, cloning and sequencing of near full-length HIV-1 subtype C genomes, J. of Virological Methods, 136 (2006) 118-125; or by Kemal et al. Methods for viral RNA isolation and PCR amplification for sequencing of near full-length HIV-1 genomes in HIV Protocols, second edition Methods in Molecular Biology vol. vol. 485, 2009, pp3-14.

Thus, according to a feature, the method of production comprises the preparation of a plasmid or cloning vector harboring an infectious lentivirus molecular clone, such as from a previously cloned virus, e.g. available in a biobank or isolated from a lentivirus infected individual or cell, or the preparation of plasmids or cloning vectors harboring infectious lentivirus molecular clones prepared from the quasi species population of lentivirus that infect a patient.

Alternatively, one can prepare inactivated Virus like particles (VLPs) instead of inactivated full length virus and use these inactivated VLPs as active principle for an immunogenic composition or a vaccine according to the invention. These inactivated VLPs can be prepared by co-transfection of suitable producer cells with a plasmid harbouring lentiviral, e.g. HIV, proviral DNA construct that does not express its envelope gene, e.g. either by stop codon mutation or deletion, together with a plasmid encoding an exogenous viral envelope such as that of the vesicular stomatitis virus protein G VSVG. A common plasmid may also be used. The transfection produces the producer cells that will be used in the rest of the process for producing inactivated virus. In the present application, these inactivated VLPs will sometimes be defined as being inactivated virus for sake of simplicity.

Alternatively, inactivated autologous HIV primary isolate to be used for vaccine purposes can also be prepared by treating HIV-infected cells with inactivating compounds subject of the invention, and directly harvesting inactivated autologous HIV released from these treated infected cells. These HIV-infected cells include but are not limited to Peripheral Blood Mononuclear Cells (PBMCs) from infected patients co-cultured or not with PBMCs from subjects not infected by HIV.

PBMCs from HIV-infected subjects and autologous HIV primary strains from these subjects can be prepared as described in Gil C et al. Vaccine. 2011 Aug 5, 29(34):5711-24. In short, CD4-enriched PBMCs from HIV-negative subjects or from HIV-infected subjects obtained by ficoll centrifugation are CD8-depleted, co-cultured and stimulated by a cocktail of anti-CD3 antibodies + IL2, in the presence of inactivating compounds at effective concentration. After several days of co-culture, half of the volume of cell supernatant is replaced by fresh medium and the cell culture is fed by fresh pre-activated CD4-enriched PBMCs from a new HIV-negative donor, still in the presence of the same effective concentration of inactivating compound. The procedure can be repeated. Autologous virus released in the supernatants and that have been inactivated in the presence of inactivating compound are isolated, analyzed for their p24 content and their absence of infectivity, and stored at -80°C.

According to a feature, a molecular characterization of the HIV clone(s) is performed. This characterization is preferably performed by full length DNA sequencing.

According to a feature, a pre-constituted plasmid is used.

The method of production then comprises the transfection of producer cell lines, preferably of human origin, such as 293T or Hela, with these cloned plasmids harboring these HIV infectious clones. Various transfection methods and transfection reagents can be used according standard Molecular Biology protocols and manufacturer's instructions. A producer cell is obtained. As an alternative, a pre-constituted producer cell is used, such as HeLa-LAV.

In short, the producer cell is cultured in the presence of an active concentration of a compound according to the invention. The culture leads to produce and release in the extracellular medium inactivated HIV virus that has lost their infectivity. The person skilled in the art may determine easily the time between transfection and addition of the inactivating agent, and the time for the cell to produce inactivated virus in the supernatant.

According to a feature, the method of production comprises the binding of the ARV agent to the LEDGF-binding pocket on lentivirus integrase, especially HIV integrase.

According to a feature, the method of production comprises the detection of an inactivated integrase multimer resulting from a shift toward higher order oligomerisation, preferably an inactived integrase tetramer of about 130 KD MW, that can be detected by cross linking experiment, by Fluorescence energy transfer (FRET) or using the method of size exclusion chromatography preferably on a Superdex PC 3.2/30 column (GE Healthcare).

The method of production may comprise a check of the absence of infectivity of these inactivated virus. This check may be performed by using target human CD4+ cells for HIV infection harboring or not reporter gene for HIV infection such as but not limited to MT4, MT2, Jurkat, TZM cell lines. According to a feature, the method thus comprises further the step of checking the absence or level of infectivity of the lentivirus.

According to a feature, the method of production comprises a step of recovering the inactivated lentivirus, especially HIV, or the VLPs, from the extracellular medium.

According to a feature, the method of production comprises further the step of purifying the inactivated lentivirus, especially HIV, or the VLPs.

The method of production may comprise the purification of the inactivated lentivirus, especially HIV virus, or VLPs preparation. The purification may be performed using standard virological and GLP procedures (Human retrovirus Protocols, Methods in Molecular Biology vol. 304, 2005, pp387-398; Retroviruses Coffin JM, Hughes SH, Varmus HE ed., Cold Spring Harbor (NY): Cold Spring Harbor Laboratory Press; 1997).

According to a feature, a pool of inactivated virus and/or VLPs preparations is done, in order to associate in the same composition several (two or more) strains.

According to a feature, the method of production comprises further the step of formulating the purified lentivirus or VLPs in a pharmaceutically acceptable carrier or vehicle, in particular one suitable for parenteral, oral, nasal or mucosal route.

The inactivated virus or VLP preparation, optionally and preferably purified, optionally pooled, is formulated. Formulation comprises mixing the inactivated virus or the inactivated VLPs with a pharmaceutically acceptable carrier or vehicle and/or an adjuvant. Preferably, the formulation comprises mixing the inactivated virus or the inactivated VLPs with a pharmaceutically acceptable carrier or vehicle and an adjuvant. Various formulations of these purified inactivated HIV virus preparations comprising one or several HIV inactivated molecular clones, together with an appropriate carrier or vehicle, preferably an adjuvant, are provided for as vaccine preparations for parenteral, mucosal, nasal or oral route, e.g. parenteral administration, such as subcutaneous injection. The composition of the vaccine will be formulated with pharmaceutically acceptable carriers or vehicles suitable for the route (Jeffery et al. Pharm. Res. (1993) 10, 362-368).

According to a feature, the method comprises the formulation of said inactivated lentivirus or VLPs with about 10⁸ to about 10¹⁰ inactivated lentivirus, or inactivated VLP, particles per ml.

According to a feature, the method of production comprises providing dendritic cells and having the dendritic cells stimulated by the inactivated lentivirus or VLPs, expecially stimulated by loading with the inactivated lentivirus or VLPs. The invention particularly includes the preparation of a dendritic cell-based vaccine with autologous monocyte-derived dendritic cells loaded with autologous inactivated viruses or VLPs (see infra).

The inactivated virus and the VLPs according to the invention can be used as an active principle of a preventive vaccine for immunization of individual that are not infected with HIV. Preferably the vaccine is used in combination with ARV drugs, in particular with the compound used to inactivate the virus or the VLPs as mean of pre-exposure prophylactic treatment.

Another possibility is that such inactivated virus or inactivated VLP can be used as an active principle of a therapeutic vaccine promoting immunotherapy for HIV-infected individuals. Preferably the vaccine is used in combination with classical ARV therapy. In such case, inactivated autologous viruses isolated from an HIV-infected individual according the methods mentioned above may advantageously be used to prepare a therapeutic vaccine as mentioned above, advantageously a dendritic cell-based vaccine loaded with autologous inactivated viruses according to the invention, in particular a dendritic cell-based vaccine with autologous monocyte-derived dendritic cells loaded with autologous inactivated viruses or VLPs according to the invention. To this aim, one may refers to methods described in García F et al., Sci Transl Med. 2013 Jan 2, 5(166):166ra2 ; García Fet al., Hum Vaccin Immunother. 2012 May, 8(5):569-81. ; Peña J et al., Viral Immunol. 2012, 25(1):37-44.; or in García F, Routy JP, Vaccine. 2011, 29(38):6454-63 ; Lu W et al., Cell Rep. 2012 Dec 27, 2(6):1736-46 ; Andrieu JM, Lu W., J Intern Med. 2007, 261(2):123-31.; Lu W et al., Nat Med. 2004,10(12):1359-65; Whiteside TL et al., Clin. Vaccine Immunol. 2009, 16(2):233-40. A particular application is purging the latent pool of HIV and thereby eradicating HIV infection.

Still another possibility is that the inactivated virus or the inactivated VLPs according to the invention is used as an active principle of a post-exposure vaccine for immunization of an individual at risk of having been exposed to HIV, this immunization being combined with a Postexposure prophylaxis treatment for HIV infection.

An object of the invention is thus an immunogenic composition or vaccine comprising an inactivated lentivirus, especially inactivated HIV, or inactivated VLPs, in a pharmaceutically acceptable carrier or vehicle, and optionally an adjuvant. The vaccine may be therapeutic or preventive. Preferably, the composition comprises a pharmaceutically acceptable carrier or vehicle and an adjuvant.

An object of the invention is especially a immunogenic composition or vaccine comprising an inactivated lentivirus, especially inactivated HIV, or inactivated VLPs, in a pharmaceutically acceptable carrier or vehicle, and optionally an adjuvant, wherein the lentivirus or the VLP comprises an inactivated integrase multimer resulting from a shift toward higher order oligomerisation preferably an inactived integrase tetramer of about 130 KD MW, that can be detected by cross linking, FRET or size exclusion chromatography. Preferably, the composition comprises a pharmaceutically acceptable carrier or vehicle and an adjuvant. The vaccine may be therapeutic or preventive.

According to a feature, the immunogenic composition or vaccine comprises dendritic cells stimulated by loading with the inactivated lentivirus or the inactivated VLPs. The invention particularly includes dendritic cell-based composition or vaccine with autologous monocyte-derived dendritic cells loaded with autologous inactivated viruses or VLPs.

According to a feature, the immunogenic composition or vaccine comprises about 10⁸ to about 10¹⁰ inactivated lentivirus or VLP particles per ml.

The pharmaceutically acceptable carrier or vehicle and the adjuvants are adapted to the route of administration, which may be in particular parenteral, mucosal, nasal or oral route. Pharmaceutically acceptable carrier or vehicle and adjuvants that can be used in the invention are described supra. These are examples and the person skilled in the art may select other candidates.

In an embodiment, the immogenic composition or vaccine comprises further an antiretroviral drug, preferably an ARV agent which is an inhibitor of the IN-LEDGF/p75 interaction.

Another object of the invention is a pharmaceutical composition or a kit for prophylactic or therapeutic treatment of a mammal, especially a human, against a lentivirus, especially HIV, comprising an immunogenic composition or vaccine according to the invention and an antiretroviral drug, preferably an ARV agent which is an inhibitor of the IN-LEDGF/p75 interaction, for a simultaneous, separate or sequential administration of the immunogenic composition or vaccine and the antiretroviral drug. The immunogenic composition or vaccine may be produced or constituted as recited herein. According to a feature, the inactivated lentivirus, in particular HIV, or the inactivated VLP, comprises a multimer of integrase having an oligomerisation state shifted toward higher molecular weight when compared to integrase from untreated infectious lentiviruses. According to a feature, the inactivated lentivirus, in particular HIV, or the inactivated VLP, comprises an inactivated integrase having an oligomerisation state shifted toward higher order multimerization, in particular an inactive integrase tetramer of 130 KD MW as estimated using the methods of cross linking experiment, FRET or size exclusion chromatography preferably on a Superdex PC 3.2/30 column (GE Healthcare).

Another object of the invention is a method of prophylactic or therapeutic treatment of a mammal, especially a human, against a lentivirus, especially HIV, such as HIV-1 and HIV-2, comprising administering an effective amount of an immunogenic composition or vaccine according to the invention. The composition or vaccine may be produced or constituted as recited herein.

In an embodiment, the method is a prophylactic method comprising the administration to an individual that is not infected with the lentivirus, e.g. HIV.

In another embodiment, the method is a therapeutic method comprising the administration to a lentivirus-infected individual, e.g. an HIV-infected individual. This includes the administration of a dendritic cell-based vaccine loaded with autologous inactivated viruses or VLPs according to the invention, in particular a dendritic cell-based vaccine with autologous monocyte-derived dendritic cells loaded with autologous inactivated viruses or VLPs according to the invention.

In still another embodiment, the method is a prophylactic method comprising the administration to an individual which is at risk of having been exposed, or that has been exposed to a lentivirus, e.g. HIV. This is a postexposure prophylaxis for lentivirus, e.g. HIV infection.

According to a feature of these methods, the method comprises the combined administration of said effective amount of an immunogenic composition or vaccine and of an effective amount of an antiretroviral drug, preferably an ARV agent which is an inhibitor of the IN-LEDGF/p75 interaction. According to a feature, the inactivated lentivirus, in particular HIV, or the inactivated VLP, comprises a multimer of integrase having an oligomerisation state shifted toward higher molecular weight. According to a feature, the inactivated lentivirus, in particular HIV, or the inactivated VLP, comprises an inactivated integrase multimer resulting from a shift toward higher order oligomerisation, preferably an inactived integrase tetramer of about 130 KD MW, that can be detected by cross linking experiment, by Fluorescence energy transfer (FRET) or using the method of size exclusion chromatography preferably on a Superdex PC 3.2/30 column (GE Healthcare).

According to a feature, the method comprises the administration of two or more doses of said immunogenic composition or vaccine.

According to a feature, the method comprises the combined administration of said effective amount of an immunogenic composition or vaccine according to the invention, and of a DNA vaccine, or a subunit vaccine, in a prime-boost combination. In an embodiment, the vaccine of the invention is used as the prime. In another embodiment, it is used as the boost.

The invention thus also relates to a pharmaceutical composition or a kit for prophylactic or therapeutic treatment of a mammal, especially a human, against a lentivirus, especially HIV, comprising an immunogenic composition or vaccine according to the invention and of a DNA vaccine, or a subunit vaccine, for a prime-boost administration of the immunogenic composition or vaccine and the DNA vaccine or the subunit vaccine. In an embodiment, the vaccine of the invention is used as the prime. In another embodiment, it is used as the boost.

According to a feature, the method comprises the combined administration of said effective amount of an immunogenic composition or vaccine according to the invention,and non-neutralizing or (broadly) neutralizing antibodies capable of inhibiting circulating viruses, and inducing a protection by passive immunization.

The invention thus also relates to a pharmaceutical composition or a kit for prophylactic or therapeutic treatment of a mammal, especially a human, against a lentivirus, especially HIV, comprising an immunogenic composition or vaccine according to the invention and non-neutralizing or (broadly) neutralizing antibodies capable of inhibiting circulating viruses, for a simultaneous, separate or sequential administration of the immunogenic composition or vaccine and the antibodies.

According to a feature, the method comprises the combined administration of said immunogenic composition or vaccine according to the invention and antiretroviral treatment (HAART) according the protocols used for pre- or postexposure prophylactic (PrEP) treatments, preferably those used for 28 days and/or no more than 90 days (R.J. Landovitz and J.S.Currier 2009, The New England Journal of Medicine, 361; 18, p1768-1775).

The methods of treatment according to the invention comprise the administration via a suitable route, which may be parenteral, mucosal, nasal or oral route. Parenteral route may encompass subcutaneous, intradermal, intramuscular, intraperitoneal and intraveinous routes, for example.

In accordance with another aspect of the invention, the inactivated lentiviruses, especially HIV, the inactivated VLPs, are used to generate antisera or antibodies. To this aim, these antigens are administered to an animal, such as rabbit, mice, rat, sheep, a non-human primate, and an antisera or antibodies is/are collected, possibly purified.

The antisera or the antibodies may be used for active principle in a pharmaceutical composition.

The following discloses inactivating compounds that can be used according to the invention to produce the inactivated virus or the inactivated VLPs.

The compounds according to the invention can be prepared according to the disclosures of patent applications EP 2 511 273, WO 2013/140243, EP 12306244.0, EP 2 508 511, WO 2012/137181, EP 12187528.0 and EP 12306222.6.

The invention provides a compound of formula (1) or (2) : 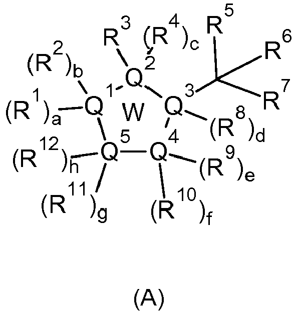 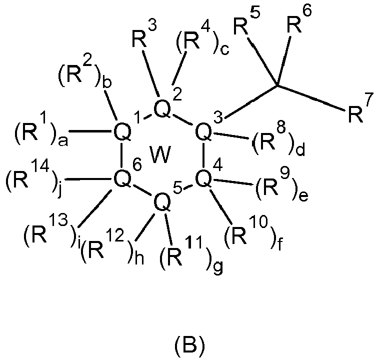 wherein :
- W represents a substituted or non-substituted, partially or totally unsaturated, aromatic or non-aromatic carbo- or heterocycle ;
- a, b, c, d, e, f, g, h, i and j independently represent 0 or 1;
- Q¹ represents CR¹, CR², CR¹R², N, NR¹,NR²,S, O, C=O, C=S, S=O, S(O)₂;
- Q² represents CR³,CR⁴, CR³R⁴, NR³, NR⁴;
- Q³ represents CR⁸(CR⁵R⁶R⁷), CR⁸(R⁸CR⁵R⁶R⁷), N(CR⁵R⁶R⁷);*
- Q⁴ represents CR⁹, CR¹⁰, CR⁹R¹⁰, N, NR⁹, NR¹⁰, S, O, C=O, C=S, S=O, S(O)₂;
- Q⁵ represents CR¹¹, CR¹², CR¹¹R¹², N, NR¹¹, NR¹², S, O, C=O, C=S, S=O, S(O)₂;
- Q⁶ represents CR¹³, CR¹⁴, CR¹³R¹⁴, N, NR¹³, NR¹⁴, S, O, C=O, C=S, S=O, S(O)₂;
- R¹, R², R⁹, R¹⁰, R¹¹, R¹², R¹³ and R¹⁴, non-substituted or substituted by at least one T¹, independently represent a hydrogen atom, -CN, -OH, -O-cycloalkyl, -O-cycloalkenyl, -O-cycloalkynyl, -NH₂, -NR¹⁵-cycloalkyl, -NR¹⁵-cycloalkenyl, -NR¹⁵-cycloalkynyl, -S-cycloalkyl, -S-cycloalkenyl, -S-cycloalkynyl, -COOR¹⁵, -OC(O)R¹⁶ , -C(O)NR¹⁵R¹⁶, -NR¹⁶C(O)R¹⁵, -CF₃, -SO₃R¹⁵, -SO₂NR¹⁵R¹⁶, -NR¹⁶SO₂R¹⁵-NR¹⁶SO₂NR¹⁵R¹⁶, -NR¹⁶C(O)NR¹⁵R¹⁶, -OC(O)NR¹⁵R¹⁶, - NR¹⁶C(O)O, halogen, alkyl, alkenyl, alkynyl, heteroalkyl, heteroalkenyl, heteroalkynyl, fluoroalkyl, fluoroalkenyl, fluoroalkynyl, aryl, -O-aryl, -NR¹⁵-aryl, -S-aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, -O-heterocycle, -NR¹⁵-heterocycle, -S-heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl; wherein a carbon atom or a heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl can be oxidized to form a C=O, C=S, S=O, S(O)₂ or S(O)₃H;
   wherein the aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, or heterocyclyl-heteroalkynyl group can be fused with at least one further cycle ;
   and wherein the alkyl, alkenyl, alkynyl, arylalkyl, arylalkenyl, arylalkynyl, cycloalkyl, cycloalkenyl, cycloalkynyl, heterocyclyl-alkyl, heterocyclyl-alkenyl, heterocyclyl-alkynyl group can include one or more heteroatoms, selected from O, S and N, within the alkyl, alkenyl, alkynyl moiety ;
   R³, non-substituted or substituted by at least one T¹, represents -O-cycloalkyl, -O-cycloalkenyl, -O-cycloalkynyl, -NR¹⁵-cycloalkyl, -NR¹⁵-cycloalkenyl, -NR¹⁵-cycloalkynyl, -S-cycloalkyl, -S-cycloalkenyl, -S-cycloalkynyl, alkyl comprising at least 4 carbon atoms, alkenyl comprising at least 4 carbon atoms, alkynyl comprising at least 4 carbon atoms, heteroalkyl, heteroalkenyl, heteroalkynyl, aryl, -O-aryl, -NR¹⁵-aryl, -S-aryl,arylalkyl, arylalkenyl, arylalkynyl, arylheteroalkyl, arylheteroalkenyl, arylheteroalkynyl, cycloalkyl, cycloalkenyl, cycloalkynyl, heterocycle, -O-heterocycle, -NR¹⁵-heterocycle, -S-heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl; wherein a carbon atom or heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, heterocycle, heterocyclyl-alkyl, heterocylyl-alkenyl, heterocyclyl-alkynyl, heterocyclyl-heteroalkyl, heterocylyl-heteroalkenyl, heterocyclyl-heteroalkynyl can be oxidized to form a C=O, C=S, S=O, S(O)₂ or S(O)₃H;
   wherein the aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, or heterocyclyl-heteroalkynyl can be fused with at least one further cycle;
   and wherein alkyl, alkenyl, alkynyl, arylalkyl, arylalkenyl, arylalkynyl, cycloalkyl, cycloalkenyl, cycloalkynyl, heterocyclyl-alkyl, heterocyclyl-alkenyl, heterocyclyl-alkynyl group can include one or more heteroatoms, selected from O, S and N, in the alkyl, alkenyl, alkynyl moiety ;
- R⁴ represents a hydrogen atom, -OH, alkyl, alkenyl, alkynyl, O-alkyl, O-alkenyl, O-alkynyl, aryl, cycloalkyl, heterocycle;
   wherein alkyl, alkenyl, alkynyl or heterocycle group can include one or more heteroatoms, selected from O, S and N ;
   wherein a carbon atom or heteroatom of said alkyl, alkenyl, alkynyl or heterocycle group, can be oxidized to form a C=O, C=S, S=O, S(O)₂ or S(O)₃H;
- R⁵ or R⁶, non-substituted or substituted by at least one T², identical, or different, independently represent a hydrogen atom, halogen, -CN, -O-cycloalkyl, -O-cycloalkenyl, -O-cycloalkynyl, -NR¹⁵-cycloalkyl, -NR¹⁵-cycloalkenyl, -NR¹⁵-cycloalkynyl, -S-cycloalkyl, -S-cycloalkenyl, -S-cycloalkynyl, alkyl, alkenyl, alkynyl, heteroalkyl, heteroalkenyl, heteroalkynyl, aryl, -O-aryl, -NR¹⁵-aryl, -S-aryl,arylalkyl, arylalkenyl, arylalkynyl, arylheteroalkyl, arylheteroalkenyl, arylheteroalkynyl, cycloalkyl, cycloalkenyl, cycloalkynyl, heterocycle, -O-heterocycle, -NR¹⁵-heterocycle, -S-heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl;
   wherein a carbon atom or heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynylcan be oxidized to form a C=O, C=S, S=O, S(O)₂ or S(O)₃H;
   wherein alkyl, alkenyl, alkynyl, arylalkyl, arylalkenyl, arylalkynyl, cycloalkyl, cycloalkenyl, cycloalkynyl, heterocyclyl-alkyl, heterocyclyl-alkenyl, heterocyclyl-alkynyl group can include one or more heteroatoms, selected from O, S and N, in the alkyl, alkenyl, alkynyl moiety;
   R⁵ and R⁶ form with the carbon atom to which they are bonded, a 3- to 7-membered carbocycle or heterocycle,
   wherein the carbocycle or heterocycle is fused with at least one further cycle, or R⁵ and R⁶ form a group of formula (i) 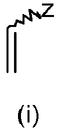 wherein Z represents a hydrogen atom, alkyl or heteroalkyl and wherein a carbon atom or heteroatom of said alkyl, can be oxidized to form a C=O, C=S, S=O, S(O)₂ or S(O)₃H ;
- R⁷ represents independently -C(O)OH, -CN, -C(O)NH₂, -C(O)OR¹⁵, -C(O)NHCN, - C(O)NHOH, -S(O)₂OH, -S(O)₂NHR¹⁵, -P(O)(OH)NH₂, -P(O)(OH)O-alkyl, -P(O)(O-alkyl)₂, -P(O)(OH)₂, -OSO₃H, -NR¹⁵SO₃H, a tetrazolyl group;
- R³ represents a hydrogen atom, alkyl, alkenyl, alkynyl;
- R¹⁵, identical or different, independently represents a hydrogen atom, alkyl, alkenyl, alkynyl, haloalkyl, haloalkenyl, haloalkynyl, cycloalkyl,cycloalkenyl,aryl, heterocycle, alkylcycloalkyl, alkylcycloalkenyl, alkenylcycloalkyl, alkynylcycloalkyl, alkenylcycloalkenyl, heterocycle, alkylheterocycle, alkenylheterocycle, alkynylheterocycle
   wherein a carbon atom of said alkyl or aryl can be oxidized to form a C=O, C=S;
- R¹⁶, identical or different, independently represents a hydrogen atom, alkyl, alkenyl, alkynyl, haloalkyl, haloalkenyl, haloalkynyl, cycloalkyl,cycloalkenyl,aryl, heterocycle, alkylcycloalkyl, alkylcycloalkenyl, alkenylcycloalkyl, alkynylcycloalkyl, alkenylcycloalkenyl, heterocycle, alkylheterocycle, alkenylheterocycle, alkynylheterocycle,;
   wherein a carbon atom of said alkyl or aryl can be oxidized to form a C=O, C=S;
- R¹⁵ and R¹⁶ form, with the azote atom to which they are bonded, a heterocycle comprising at least one N atom;
- T¹, identical or different, independently represents a hydrogen atom, halogen,-OT³, -OCF₃, =O, -ST³, =S, -S(O)T⁴, -S(O)₂T⁴, -S(O)₂NT⁵T⁶, CF₃, NO₂, -NT⁵T⁶,-NT³S(O)₂T⁴, CN, -NT³C(O)T⁴, -NT³C(O)NT⁵T⁶, -C(O)OT³, -C(O)NT⁵T⁶, -C(O)T⁴, alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl,heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl:
   wherein said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl,heterocycle, heterocyclyl-alkyl, heterocylyl-alkenyl, heterocyclyl-alkynyl, heterocyclyl-heteroalkyl, heterocylyl-heteroalkenyl, heterocyclyl-heteroalkynyl can be substituted with one or more T⁷;
   wherein a carbon atom or heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl,heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl can be oxidized to form a C=O, C=S, S=O, S(O)₂ or S(O)₃H ;
- T², identical or different, independently represents a hydrogen atom, halogen,-OT⁸, -OCF₃, =O, -ST⁸, =S, -S(O)T⁹, -S(O)₂T⁹, -S(O)₂NT¹⁰T¹¹, -CF₃, -NO₂, -NT¹⁰T¹¹, -NT⁸S(O)₂T⁹, -CN, -NT⁸C(O)T⁹, -NT⁸C(O)NT¹⁰T¹¹, -C(O)OT⁸, -C(O)NT¹⁰T¹¹,-C(O)T⁹, alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl,heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl;
   wherein said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl,heterocycle, heterocyclyl-alkyl, heterocylyl-alkenyl, heterocyclyl-alkynyl, heterocyclyl-heteroalkyl, heterocylyl-heteroalkenyl, heterocyclyl-heteroalkynyl can be substituted with one or more T⁷;
   wherein a carbon atom or heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl can be oxidized to form a C=O, C=S, S=O, S(O)₂ or S(O)₃H ;
- T³, identical or different, independently represents a hydrogen atom, alkyl, alkenyl, alkynyl, heteroalkyl, heteroalkenyl, heteroalkynyl, aryl, heterocycle;
   wherein said alkyl, alkenyl, alkynyl, heteroalkyl, heteroalkenyl, heteroalkynyl, aryl, heterocycle can be substituted or non substituted with one or more -OH, =O, halogen, -SH, =S, -CF₃, -O-alkyl, -OCF₃, -CN, -NO₂, -C(O)OH, -NH₂ or C(O)NH₂; wherein a carbon atom or heteroatom of said alkyl, alkenyl, alkynyl, heteroalkyl, heteroalkenyl, heteroalkynyl, aryl, heterocycle can be oxidized to form a C=O, C=S, S=O, S(O)₂ or S(O)₃H ;
- T⁴, identical or different, independently represents -OH, alkyl, alkenyl, alkynyl, heteroalkyl, heteroalkenyl, heteroalkynyl, aryl, heterocycle;
   wherein said alkyl, alkenyl, alkynyl, heteroalkyl, heteroalkenyl, heteroalkynyl, aryl, heterocycle, can be substituted or non substituted with one or more -OH, =O, halogen, -SH, =S, -CF₃, -O-alkyl, -OCF₃, -CN, -NO₂, -C(O)OH, -NH₂ or C(O)NH₂; wherein a carbon atom or heteroatom of said alkyl, alkenyl, alkynyl, heteroalkyl, heteroalkenyl, heteroalkynyl aryl, heterocycle can be oxidized to form a C=O, C=S, S=O,S(O)₂ or S(O)₃H;
- T⁵ or T⁶, identical or different, independently represent a hydrogen atom, alkyl, alkenyl, alkynyl, heteroalkyl, heteroalkenyl, heteroalkynyl, aryl, heterocycle wherein said alkyl, alkenyl, alkynyl, heteroalkyl, heteroalkenyl, heteroalkynyl, aryl, heterocycle can be substituted or non substituted with one or more -OH, =O, halogen, -SH, =S, -CF₃, -O-alkyl, -OCF₃, -CN, -NO₂, -C(O)OH, -NH₂ or C(O)NH₂; wherein a carbon atom or heteroatom of said alkyl, alkenyl, alkynyl, heteroalkyl, heteroalkenyl, heteroalkynyl aryl, heterocycle can be oxidized to form a C=O, C=S, , S=O, S(O)₂ or S(O)₃H ;
   or T⁵ or T⁶ can be form, with the azote atom to which they are bonded, a 4-, 5-, 6-or 7- membered heterocycle non substituted or substituted with an alkyl, alkenyl, alkynyl, heteroalkyl, heteroalkenyl, heteroalkynyl, -OH, halogen, -SH, -CF₃, -O-alkyl, -OCF₃, -CN, -NO₂, -C(O)OH, -NH₂ or -C(O)NH₂;
- T⁷, identical or different, independently represents an alkyl, alkenyl, alkynyl, heteroalkyl, heteroalkenyl, heteroalkynyl, -OH, =O, halogen, -SH, =S, -CF₃, -CN, - NO₂, -C(O)OH, -NH₂ or -C(O)NH₂;
- T⁸, identical or different, independently represents a hydrogen atom, alkyl, alkenyl, alkynyl, heteroalkyl, heteroalkenyl, heteroalkynyl;
   wherein said alkyl, alkenyl, alkynyl, heteroalkyl, heteroalkenyl, heteroalkynyl, can be substituted or non substituted with one or more -OH, =O, halogen, -SH, =S,-CF₃, -O-alkyl, -OCF₃, -CN, -NO₂, -C(O)OH, -NH₂ or -C(O)NH₂;
   wherein a carbon atom or heteroatom of said alkyl, alkenyl, alkynyl, heteroalkyl, heteroalkenyl, heteroalkynyl can be oxidized to form a C=O, C=S, S=O, S(O)₂ or S(O)₃H ;
- T⁹, identical or different, independently represents -OH, alkyl, alkenyl, alkynyl, heteroalkyl, heteroalkenyl, heteroalkynyl, wherein said alkyl, alkenyl, alkynyl, heteroalkyl, heteroalkenyl, heteroalkynyl, can be substituted or non substituted with one or more -OH, =O, halogen, -SH, =S, -CF₃, -O-alkyl, -OCF₃, -CN, -NO₂, - C(O)OH, -NH₂ or -C(O)NH₂;
   wherein a carbon atom or heteroatom of said alkyl, alkenyl, alkynyl, heteroalkyl, heteroalkenyl, heteroalkynyl can be oxidized to form a C=O, C=S, S=O, S(O)₂ or S(O)₃H ;
- T¹⁰ or T¹¹, identical or different, independently represent a hydrogen atom, alkyl, alkenyl, alkynyl, heteroalkyl, heteroalkenyl, heteroalkynyl;
   wherein said alkyl, alkenyl, alkynyl, heteroalkyl, heteroalkenyl, heteroalkynyl, can be substituted or non substituted with one or more -OH, =O, halogen, -SH, =S, - CF₃, -O-alkyl, -OCF₃, -CN, -NO₂, -C(O)OH, -NH₂ or -C(O)NH₂;
   wherein a carbon atom or heteroatom of said alkyl, alkenyl, alkynyl, heteroalkyl, heteroalkenyl, heteroalkynyl can be oxidized to form a C=O, C=S, S=O, S(O)₂ or S(O)₃H;
   or T¹⁰ or T¹¹ can be form, with the azote atom to which they are bonded, a 4-, 5-, 6-or 7- membered heterocycle non substituted or substituted with an alkyl, alkenyl, alkynyl, heteroalkyl, heteroalkenyl, heteroalkynyl, -OH, halogen, -SH, -CF₃, O-alkyl, -OCF₃, -CN, -N02, -C(O)OH, -NH₂ or -C(O)NH₂;
and a racemate, enantiomer, isomer or diastereoisomer or a phamaceutically acceptable salt thereof.

The invention also provides a compound of formula (A) or (B) wherein:
- R³ and R⁴ form with Q² a saturated, partially or totally unsaturated 5-, 6- or 7-membered carbocycle or heterocycle or a saturated, partially or totally unsaturated 7-, 8-, 9-, 10-, 11-, 12-, 13- or 14-membered polycarbo- or polyheterocycle, non-substituted or substituted by at least one T¹;
   wherein the heterocycle comprises at least one heteroatom selected from O, N or S;
   wherein a carbon atom or heteroatom of said carbo- or heterocycle can be oxidized to form a C=O, C=S, S=O, S(O)₂ or S(O)₃H;
   wherein the carbo- or heterocycle can be fused with at least one further cycle;
- W, a, b, d, e, f, g, h, i, j, Q¹, Q³, Q⁴, Q⁵, Q⁶, R¹, R², R⁵, R⁶, R⁷, R⁸, R⁹, R¹⁰, R¹¹, R¹², R¹³,R¹⁴, R¹⁵, R¹⁶, T¹, T², T³, T⁴, T⁵, T⁶, T⁷, T⁸, T⁹, T¹⁰ and T¹¹ are defined for compound of formula (A) or (B).

The invention also provides a compound of formula (B) wherein:
- R¹, Q¹, Q⁶ and R¹³ form a saturated, partially or totally unsaturated 5-, 6- or 7-membered carbo- or hetero-cycle or a saturated, partially or totally unsaturated 7-, 8-, 9-,10-, 11-, 12-, 13- or 14-membered polycarbo- or polyheterocycle;
- W, b, c, d, e, f, g, h, j, Q¹, Q², Q³, Q⁴, Q⁵, Q⁶, R², R³, R⁴, R⁵, R⁶, R⁷, R⁸, R⁹, R¹⁰, R¹¹, R¹², R¹⁴, R¹⁵, R¹⁶, T¹, T², T³, T⁴, T⁵, T⁶, T⁷, T⁸, T⁹, T¹⁰ and T¹¹ are defined for compound of formula (B).

The invention also provides a compound of formula (B) wherein:
- R¹, Q¹, Q⁶ and R¹⁴ form a saturated, partially or totally unsaturated 5-, 6- or 7-membered carbocycle or heterocycle or a saturated, partially or totally unsaturated 7-, 8-, 9-,10-, 11-, 12-, 13- or 14-membered polycarbocycle or polyheterocycle;
- W, b, c, d, e, f, g, h, i, Q¹, Q², Q³, Q⁴, Q⁵, Q⁶, R², R³, R⁴, R⁵, R⁶, R⁷, R⁸, R⁹, R¹⁰, R¹¹, R¹², R¹³, R¹⁵, R¹⁶, T¹, T², T³, T⁴, T⁵, T⁶, T⁷, T⁸, T⁹, T¹⁰ and T¹¹ are defined for compound of formula (B).

The invention also provides a compound of formula (B) wherein:
- R², Q¹, Q⁶ and R¹³ form a saturated, partially or totally unsaturated 5-, 6- or 7-membered carbocycle or heterocycle or a saturated, partially or totally unsaturated 7-, 8-, 9-,10-, 11-, 12-, 13- or 14-membered polycarbocycle or polyheterocycle;
- W, a, c, d, e, f, g, h, j, Q¹, Q², Q³, Q⁴, Q⁵, Q⁶, R¹, R³, R⁴, R⁵, R⁶, R⁷, R⁸, R⁹, R¹⁰, R¹¹, R¹², R¹⁴, R¹⁵, R¹⁶, T¹, T², T³, T⁴, T⁵, T⁶, T⁷, T⁸, T⁹, T¹⁰ and T¹¹ are defined for compound of formula (B).

The invention also provides a compound of formula (B) wherein:
- R², Q¹, Q⁶ and R¹⁴ form a saturated, partially or totally unsaturated 5-, 6- or 7-membered carbocycle or heterocycle or a saturated, partially or totally unsaturated 7-, 8-, 9-,10-, 11-, 12-, 13- or 14-membered polycarbocycle or polyheterocycle;
- W, a, c, d, e, f, g, h, i, Q¹, Q², Q³, Q⁴, Q⁵, Q⁶, R¹, R³, R⁴, R⁵, R⁶, R⁷, R⁸, R⁹, R¹⁰, R¹¹, R¹², R¹³, R¹⁵, R¹⁶, T¹, T², T³, T⁴, T⁵, T⁶, T⁷, T⁸, T⁹, T¹⁰ and T¹¹ are defined for compound of formula (B).

The invention also provides a compound of formula (B) wherein:
- R¹¹, Q⁵, Q⁶ and R¹³ form a saturated, partially or totally unsaturated 5-, 6- or 7-membered carbocycle or heterocycle or a saturated, partially or totally unsaturated 7-, 8-, 9-, 10-, 11-, 12-, 13- or 14-membered polycarbocycle or polyheterocycle;
- W, a, b, c, d, e, f, h, j, Q¹, Q², Q³, Q⁴, Q⁵, Q⁶, R¹, R², R³, R⁴, R⁵, R⁶, R⁷, R⁸, R⁹, R¹⁰, R¹², R¹⁴, R¹⁵, R¹⁶, T¹, T², T³, T⁴, T⁵, T⁶, T⁷, T⁸, T⁹, T¹⁰ and T¹¹ are defined for compound of formula (B).

The invention also provides a compound of formula (B) wherein:
- R¹¹, Q⁵, Q⁶ and R¹⁴ form a saturated, partially or totally unsaturated 5-, 6- or 7-membered carbocycle or heterocycle or a saturated, partially or totally unsaturated 7-, 8-, 9-, 10-, 11-, 12-, 13- or 14-membered polycarbocycle or polyheterocycle;
- W, a, b, c, d, e, f, h, i, Q¹, Q², Q³, Q⁴, Q⁵, Q⁶, R¹, R², R³, R⁴, R⁵, R⁶, R⁷, R⁸, R⁹, R¹⁰, R¹², R¹³, R¹⁵, R¹⁶, T¹, T², T³, T⁴, T⁵, T⁶, T⁷, T⁸, T⁹, T¹⁰ and T¹¹ are defined for compound of formula (B).

The invention also provides a compound of formula (B) wherein:
- R¹², Q⁵, Q⁶ and R¹³ form a saturated, partially or totally unsaturated 5-, 6- or 7-membered carbocycle or heterocycle or a saturated, partially or totally unsaturated 7-, 8-, 9-, 10-, 11-, 12-, 13- or 14-membered polycarbocycle or polyheterocycle;
- W, a, b, c, d, e, f, g, j, Q¹, Q², Q³, Q⁴, Q⁵, Q⁶, R¹, R², R³, R⁴, R⁵, R⁶, R⁷, R⁸, R⁹, R¹⁰, R¹¹, R¹⁴, R¹⁵, R¹⁶, T¹, T², T³, T⁴, T⁵, T⁶, T⁷, T⁸, T⁹, T¹⁰ and T¹¹ are defined for compound of formula (B).

The invention also provides a compound of formula (B) wherein:
- R¹², Q⁵, Q⁶ and R¹⁴ form a saturated, partially or totally unsaturated 5-, 6- or 7-membered carbocycle or heterocycle or a saturated, partially or totally unsaturated 7-, 8-, 9-, 10-, 11-, 12-, 13- or 14-membered polycarbocycle or polyheterocycle;
- W, a, b, c, d, e, f, g, i, Q¹, Q², Q³, Q⁴, Q⁵, Q⁶, R¹, R², R³, R⁴, R⁵, R⁶, R⁷, R⁸, R⁹, R¹⁰, R¹¹, R¹³, R¹⁵, R¹⁶, T¹, T², T³, T⁴, T⁵, T⁶, T⁷, T⁸, T⁹, T¹⁰ and T¹¹ are defined for compound of formula (B).

The invention also provides a compound of formula (A) or (B) wherein:
- R⁹, Q⁴, Q⁵ and R¹¹ form a saturated, partially or totally unsaturated 5-, 6- or 7-membered carbocycle or heterocycle or a saturated, partially or totally unsaturated 7-, 8-, 9-, 10-, 11-, 12-, 13- or 14-membered polycarbocycle or polyheterocycle;
- W, a, b, c, d, f, h, i, j, Q¹, Q², Q³, Q⁴, Q⁵, Q⁶, R¹, R², R³, R⁴, R⁵, R⁶, R⁷, R⁸, R¹⁰, R¹², R¹³ R¹⁴, R¹⁵, R¹⁶, T¹, T², T³, T⁴, T⁵, T⁶, T⁷, T⁸, T⁹, T¹⁰ and T¹¹ are defined for compound of formula (A) or (B).

The invention also provides a compound of formula (A) or (B)wherein:
- R⁹, Q⁴, Q⁵ and R¹² form a saturated, partially or totally unsaturated 5-, 6- or 7-membered carbocycle or heterocycle or a saturated, partially or totally unsaturated 7-, 8-, 9-, 10-, 11-, 12-, 13- or 14-membered polycarbocycle or polyheterocycle;
- W, a, b, c, d, f, g, i, j, Q¹, Q², Q³, Q⁴, Q⁵, Q⁶, R¹, R², R³, R⁴, R⁵, R⁶, R⁷, R⁸, R¹⁰, R¹¹, R¹³, R¹⁴, R¹⁵, R¹⁶, T¹, T², T³, T⁴, T⁵, T⁶, T⁷, T⁸, T⁹, T¹⁰ and T¹¹ are defined for compound of formula (A) or (B).

The invention also provides a compound of formula (A) or (B) wherein:
- R¹⁰, Q⁴, Q⁵ and R¹¹ form a saturated, partially or totally unsaturated 5-, 6- or 7-membered carbocycle or heterocycle or a saturated, partially or totally unsaturated 7-, 8-, 9-, 10-, 11-, 12-, 13- or 14-membered polycarbocycle or polyheterocycle;
- W, a, b, c, d, e, h, i, j, Q¹, Q², Q³, Q⁴, Q⁵, Q⁶, R¹, R², R³, R⁴, R⁵, R⁶, R⁷, R⁸, R⁹, R¹², R¹³, R¹⁴, R¹⁵, R¹⁶, T¹, T², T³, T⁴, T⁵, T⁶, T⁷, T⁸, T⁹, T¹⁰ and T¹¹ are defined for compound of formula (A) or (B).

The invention also provides a compound of formula (A) or (B)wherein:
- R¹⁰, Q⁴, Q⁵ and R¹² form a saturated, partially or totally unsaturated 5-, 6- or 7-membered carbocycle or heterocycle or a saturated, partially or totally unsaturated 7-, 8-, 9-, 10-, 11-, 12-, 13- or 14-membered polycarbocycle or polyheterocycle;
- W, a, b, c, d, e, g, i, j, Q¹, Q², Q³, Q⁴, Q⁵, Q⁶, R¹, R², R³, R⁴, R⁵, R⁶, R⁷, R⁸, R⁹, R¹¹, R¹³, R¹⁴, R¹⁵, R¹⁶, T¹, T², T³, T⁴, T⁵, T⁶, T⁷, T⁸, T⁹, T¹⁰ and T¹¹ are defined for compound of formula (A) or (B).

The invention also provides a compound of formula (A) wherein:
- R¹, Q¹, Q⁵ and R¹¹ form a saturated, partially or totally unsaturated 5-, 6- or 7-membered carbo- or hetero-cycle or a saturated, partially or totally unsaturated 7-, 8-, 9-, 10-, 11-, 12-, 13- or 14-membered polycarbo- or polyheterocycle;
- W, b, c, d, e, f, h, Q¹, Q², Q³, Q⁴, Q⁵, R², R³, R⁴, R⁵, R⁶, R⁷, R⁸, R⁹, R¹⁰, R¹², R¹⁵, R¹⁶, T¹, T², T³, T⁴, T⁵, T⁶, T⁷, T⁸, T⁹, T¹⁰ and T¹¹ are defined for compound of formula (A).

The invention also provides a compound of formula (A) wherein:
- R¹, Q¹, Q⁵ and R¹² form a saturated, partially or totally unsaturated 5-, 6- or 7-membered carbocycle or heterocycle or a saturated, partially or totally unsaturated 7-, 8-, 9-, 10-, 11-, 12-, 13- or 14-membered polycarbocycle or polyheterocycle;
- W, b, c, d, e, f, g, Q¹, Q², Q³, Q⁴, Q⁵, R², R³, R⁴, R⁵, R⁶, R⁷, R⁸, R⁹, R¹⁰, R¹¹, R¹⁵, R¹⁶, T¹, T², T³, T⁴, T⁵, T⁶, T⁷, T⁸, T⁹, T¹⁰ and T¹¹ are defined for compound of formula (A).

The invention also provides a compound of formula (A) wherein:
- R², Q¹, Q⁵ and R¹¹ form a saturated, partially or totally unsaturated 5-, 6- or 7-membered carbocycle or heterocycle or a saturated, partially or totally unsaturated 7-, 8-, 9-, 10-, 11-, 12-, 13- or 14-membered polycarbocycle or polyheterocycle;
- W, a, c, d, e, f, h, Q¹, Q², Q³, Q⁴, Q⁵, R¹, R³, R⁴, R⁵, R⁶, R⁷, R⁸, R⁹, R¹⁰, R¹², R¹⁵, R¹⁶, T¹, T², T³, T⁴, T⁵, T⁶, T⁷, T⁸, T⁹, T¹⁰ and T¹¹ are defined for compound of formula (A).

The invention also provides a compound of formula (A) wherein:
- R², Q¹, Q⁵ and R¹² form a saturated, partially or totally unsaturated 5-, 6- or 7-membered carbocycle or heterocycle or a saturated, partially or totally unsaturated 7-, 8-, 9-, 10-, 11-, 12-, 13- or 14-membered polycarbocycle or polyheterocycle;
- W, a, c, d, e, f, g, Q¹, Q², Q³, Q⁴, Q⁵, R¹, R³, R⁴, R⁵, R⁶, R⁷, R⁸, R⁹, R¹⁰, R¹¹, R¹⁵, R¹⁶, T¹, T², T³, T⁴, T⁵, T⁶, T⁷, T⁸, T⁹, T¹⁰ and T¹¹ are defined for compound of formula (A).

The invention also provides a compound of formula (1A) : 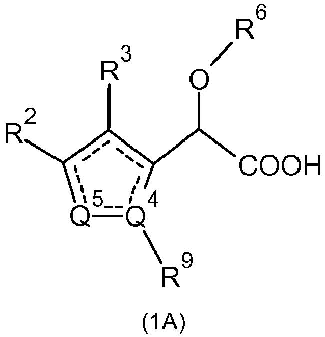 wherein :
- W represents a substituted or non-substituted, partially or totally unsaturated, aromatic or non-aromatic carbo- or heterocycle ;
- Q⁴ represents CR⁹, N, NR⁹, S, O, S=O or S(O)₂;
- Q⁵ represents N, S, O, S=O or S(O)₂;
- R⁹ represents a halogen atom; -CF₃; a linear or branched C₁-C₆ alkyl; a linear or branched C₂-C₆ alkenyl; a linear or branched C₂-C₆ alkynyl a linear or branched fluoroalkyl; a C₃-C₆ cycloalkyl, -CH₂OH or -CH₂-O-CH₃R², non-substituted or substituted by at least one T¹, represents a partially or totally unsaturated or aromatic 5-, 6- or 7-membered carbocycle ; a partially or totally unsaturated or aromatic 5-, 6- or 7-membered heterocycle; a partially or totally unsaturated or aromatic 5-, 6- or 7-membered carbocycle fused with a saturated, partially or totally unsaturated 5-, 6- or 7-membered carbocycle; a partially or totally unsaturated or aromatic 5-, 6- or 7-membered carbocycle fused with a saturated, partially or totally unsaturated 5-, 6- or 7-membered heterocycle; a partially or totally unsaturated or aromatic 5-, 6- or 7-membered heterocycle fused with a saturated, partially or totally unsaturated 5-, 6- or 7-membered carbocycle; a partially or totally unsaturated or aromatic 5-, 6- or 7-membered heterocycle fused with a saturated, partially or totally unsaturated 5-, 6- or 7-membered heterocycle;
- R², non-substituted or substituted by at least one T¹, represents a partially or totally unsaturated or aromatic 5-, 6- or 7-membered carbocycle; a partially or totally unsaturated or aromatic 5-, 6- or 7-membered heterocycle; a partially or totally unsaturated or aromatic 5-, 6- or 7-membered carbocycle fused with a saturated, partially or totally unsaturated 5-, 6- or 7-membered carbocycle; a partially or totally unsaturated or aromatic 5-, 6- or 7-membered carbocycle fused with a saturated, partially or totally unsaturated 5-, 6- or 7-membered heterocycle; a partially or totally unsaturated or aromatic 5-, 6- or 7-membered heterocycle fused with a saturated, partially or totally unsaturated 5-, 6- or 7-membered carbocycle; a partially or totally unsaturated or aromatic 5-, 6- or 7-membered heterocycle fused with a saturated, partially or totally unsaturated 5-, 6- or 7-membered heterocycle;
- R³, non-substituted or substituted by at least one T², represents an aryl; an aryl fused with a saturated, partially or totally unsaturated or aromatic 5-, 6- or 7-membered carbocycle; an aryl fused with a saturated, partially or totally unsaturated or aromatic 5-, 6- or 7-membered heterocycle; a heteroaryl; a heteroaryl fused with a saturated, partially or totally unsaturated or aromatic 5-, 6-or 7-membered carbocycle; a heteroaryl fused with a saturated, partially or totally unsaturated or aromatic 5-, 6- or 7-membered heterocycle or a C₃-C₇ cycloalkenyl;
- R⁶ represents a linear or branched C₁-C₆-alkyl; a linear or branched C₁-C₆ fluoroalkyl or a C₃-C₆ cycloalkyl;
- T¹, identical or different, independently represents a hydrogen atom , a halogen atom; an alkyl; -(X)ₐ-C₁-C₆ alkyl; a linear or branched fluorooalkyl; a linear or branched -O-C₁-C₃ fluorooalkyl; -(X)ₐ-C₃-C₆ cycloalkyl; -(X)ₐ-(CT⁵T⁶)_{b}-aryl; -(X)ₐ-(CT⁵T⁶)_{b}CN; -(X)ₐ-(CT⁵T⁶)_{b}OT³; -(X)ₐ-(CT⁵T⁶)_{b}ST³; -(X)ₐ-(CT⁵T⁶)_{b}S(O)T³; -(X)ₐ-(CT⁵T⁶)_{b}S(O)₂T³; -(X)ₐ-(CT⁵T⁶)_{b}NT³T⁴; -(X)ₐ-(CT⁵T⁶)_{b}C(O)T³; -(X)ₐ-(CT⁵T⁶)_{b}C(O)OT³; -(X)ₐ-(CT⁵T⁶)_{b}C(O)NT³T⁴; -(X)ₐ-(CT⁵T ⁶)_{b}NT³C(O)NT³T⁴; -(X)ₐ-(CT⁵T⁶)_{b}NT³C(O)T⁴; -(X)ₐ-(CT⁵T⁶)_{b}NT³C(O)OT⁴; -(X)ₐ-(CT⁵T⁶)_{b}OC(O)NT³T⁴; -(X)ₐ-(CT⁵T⁶)_{b}S(O)₂NT³T⁴ or -(X)ₐ-(CT⁵T⁶)_{b}NT³S(O)₂T⁴;
- T², identical or different, independently represents a hydrogen atom ; a halogen atom; a linear or branched -O-C₁-C₃ alkyl; a linear or branched C₁-C₃ fluoroalkyl; a linear or branched -O-C₁-C₃ fluoroalkyl; a linear or branched C₁-C₃ alkyl; cyclopropyl or -CN;
- X represents an oxygen atom; a sulphur atom; NT³; S=O or S(O)₂;
- T³ and T⁴, identical or different, independently represent H; a branched or linear C₁-C₆ alkyl or a C₃-C₆ cycloalkyl;
- T³, T⁴ and the nitrogen atom to which they are bonded form a C₄-C₆ cycloalkyl;
- T⁵ and T⁶, identical or different, independently represent a hydrogen atom ; a fluorine atom or a linear or branched C₁-C₃ alkyl or a C₃-C₆ cycloalkyl;
- T⁵, T⁶ and the carbon atom to which they are bonded form a cyclopropyl;
- a represents 0 or 1;
- b represents 0, 1, 2 or 3;
and a racemate, enantiomer, isomer, tautomer, atropoisomer or diastereoisomer or a phamaceutically acceptable salt thereof.

The invention also provides a compound of formula (1A1): 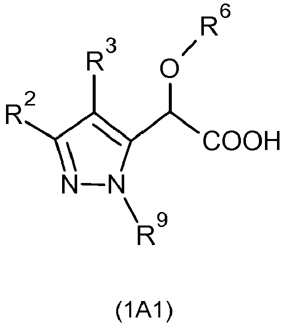 wherein R⁹, R², R³ and R⁶ are defined for compounds of formula (1A).

The invention also provides a compound of formula (1A2): 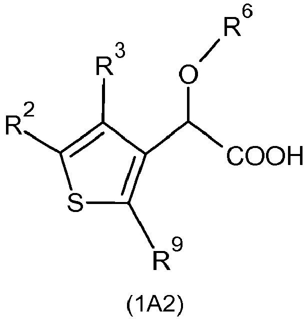 wherein R⁹, R², R³ and R⁶ are defined for compounds of formula (1A).

The invention also provides a compound of formula (1 B) : 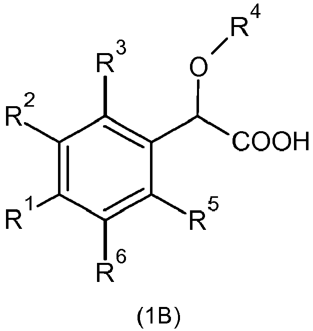 wherein :
- R¹ and R⁶, non-substituted or substituted by at least one T¹, identical or different, independently represent a hydrogen atom; -CN; -OH; -CF₃; a halogen atom; a linear or branched C₁-C₈ alkyl; a linear or branched C₂-C₈ alkenyl; a linear or branched C₂-C₈ alkynyl; -Z-C(O)OR⁷; -Z-S(O)OR⁷; -Z-OC(O)OR⁷; -Z-OR⁸; -Z-SR⁸; -Z-NR⁷R⁸; -Z-OC(O)R⁸; -Z-C(O)R⁸; -Z-C(O)NR⁷R⁸; -Z-NR⁸C(O)R⁸; -Z-OC(O)NR⁷R⁸; -Z-NR⁸C(O)OR⁷; -Z-S(O)NR⁷R⁸; a saturated, partially or totally unsaturated or aromatic 3-, 4-, 5-, 6- or 7-membered carbocycle; a saturated, partially or totally unsaturated or aromatic 3-, 4-, 5-, 6- or 7-membered carbocycle fused with a saturated, partially or totally unsaturated or aromatic 3-, 4-, 5-, 6- or 7-membered carbocycle; a saturated, partially or totally unsaturated or aromatic 5-, 6- or 7-membered carbocycle fused with a saturated, partially or totally unsaturated or aromatic 4-, 5-, 6- or 7-membered heterocycle; a saturated, partially or totally unsaturated or aromatic 4-, 5-, 6- or 7-membered heterocycle; a saturated, partially or totally unsaturated or aromatic 4-, 5-, 6- or 7-membered heterocycle fused with a saturated, partially or totally unsaturated or aromatic 3-, 4-, 5-, 6- or 7-membered carbocycle; a saturated, partially or totally unsaturated or aromatic 4-, 5-, 6- or 7-membered heterocycle fused with a saturated, partially or totally unsaturated or aromatic 4-, 5-, 6- or 7-membered heterocycle;
- R² non-substituted or substituted by at least one T¹, represents a linear or branched C₂-C₈ alkyl; a linear or branched C₂-C₈ alkenyl; a linear or branched C₁-C₈ heteroalkyl; a linear or branched C₂-C₈ heteroalkenyl; a C₃-C₇ cycloalkyl; a partially or totally unsaturated or aromatic 4-, 5-, 6- or 7-membered carbocycle ; a partially or totally unsaturated or aromatic 4-, 5-, 6- or 7-membered heterocycle; a partially or totally unsaturated or aromatic 5-, 6- or 7-membered carbocycle fused with a saturated, partially or totally unsaturated 5-, 6- or 7-membered carbocycle; a partially or totally unsaturated or aromatic 5-, 6- or 7-membered carbocycle fused with a saturated, partially or totally unsaturated 5-, 6- or 7-membered heterocycle; a partially or totally unsaturated or aromatic 5-, 6- or 7-membered heterocycle fused with a saturated, partially or totally unsaturated 5-, 6- or 7-membered carbocycle; a partially or totally unsaturated or aromatic 5-, 6- or 7-membered heterocycle fused with a saturated, partially or totally unsaturated 5-, 6- or 7-membered heterocycle; a C₁-C₈ alkyl-(C₃-C₇ cycloalkyl); a C₁-C₈ heteroalkyl-(C₃-C₇ cycloalkyl); a C₁-C₈ alkyl-(partially or totally unsaturated or aromatic C₃-C₇ carbocycle); a C₁-C₈ heteroalkyl-(partially or totally unsaturated or aromatic C₃-C₇ carbocycle); a C₄-C₇ heterocycloalkyl; a C₁-C₈ alkyl-(C₄-C₇ heterocycloalkyl); a C₁-C₈ heteroalkyl-(C₄-C₇ heterocycloalkyl); a C₁-C₈ alkyl-(partially or totally unsaturated or aromatic C₄-C₇ heterocycle); a C₁-C₈ heteroalkyl-(partially or totally unsaturated or aromatic C₄-C₇ heterocycle);R³, non-substituted or substituted by at least one T², represents a saturated, partially or totally unsaturated or aromatic 5-, 6- or 7-membered carbocycle; a saturated, partially or totally unsaturated or aromatic 5-, 6- or 7-membered carbocycle fused with a saturated, partially or totally unsaturated or aromatic 3-, 4-, 5-, 6- or 7-membered carbocycle; a saturated, partially or totally unsaturated or aromatic 5-, 6- or 7-membered carbocycle fused with a saturated, partially or totally unsaturated or aromatic 4-, 5-, 6- or 7-membered heterocycle; a saturated, partially or totally unsaturated or aromatic 5-, 6- or 7-membered carbocycle fused with a saturated, partially or totally unsaturated or aromatic 3-, 4-, 5-, 6- or 7-membered carbocycle and further fused with a saturated, partially or totally unsaturated or aromatic 4-, 5-, 6- or 7-membered heterocycle; a saturated, partially or totally unsaturated or aromatic 5-, 6- or 7-membered carbocycle fused with a saturated, partially or totally unsaturated or aromatic 4-, 5-, 6- or 7-membered heterocycle and further fused with a saturated, partially or totally unsaturated or aromatic 4-, 5-, 6- or 7-membered heterocycle; a saturated, partially or totally unsaturated or aromatic 5-, 6- or 7-membered carbocycle fused with a saturated, partially or totally unsaturated or aromatic 3-, 4-, 5-, 6- or 7-membered carbocycle and further fused with a saturated, partially or totally unsaturated or aromatic 3-, 4-, 5-, 6- or 7-membered carbocycle; a saturated, partially or totally unsaturated or aromatic 5-, 6- or 7-membered heterocycle; a saturated, partially or totally unsaturated or aromatic 5-, 6- or 7-membered heterocycle fused with a saturated, partially or totally unsaturated or aromatic 3-, 4-, 5-, 6- or 7-membered carbocycle; a saturated, partially or totally unsaturated or aromatic 5-, 6- or 7-membered heterocycle fused with a saturated, partially or totally unsaturated or aromatic 4-, 5-, 6- or 7-membered heterocycle; a saturated, partially or totally unsaturated or aromatic 5-, 6- or 7-membered heterocycle fused with a saturated, partially or totally unsaturated or aromatic 3-, 4-, 5-, 6- or 7-membered carbocycle and further fused with a saturated, partially or totally unsaturated or aromatic 4-, 5-, 6- or 7-membered heterocycle; a saturated, partially or totally unsaturated or aromatic 5-, 6- or 7-membered heterocycle fused with a saturated, partially or totally unsaturated or aromatic 4-, 5-, 6- or 7-membered heterocycle and further fused with a saturated, partially or totally unsaturated or aromatic 4-, 5-, 6- or 7-membered heterocycle; a saturated, partially or totally unsaturated or aromatic 5-, 6- or 7-membered heterocycle fused with a saturated, partially or totally unsaturated or aromatic 3-, 4-, 5-, 6- or 7-membered carbocycle and further fused with a saturated, partially or totally unsaturated or aromatic 3-, 4-, 5-, 6- or 7-membered carbocycle;R⁴, substituted or non-substituted by at least one T³, represents a linear or branched C₁-C₆-alkyl; a linear or branched C₁-C₆ fluoroalkyl or a C₃-C₆ cycloalkyl;
- R⁵ represents a halogen atom; -CF₃; a linear or branched C₁-C₆ alkyl; a linear or branched C₂-C₆ alkenyl; a linear or branched C₂-C₆ alkynyl; a linear or branched fluoroalkyl; a C₃-C₆ cycloalkyl, -CH₂OH or -CH₂-O-CH₃;
- R⁵ and R⁶ form, with the carbon atoms to which they are bonded, an aryl or form, with the carbon atoms to which they are bonded a heteroaryl comprising at least one N atom;R⁷ and R⁸, identical or different, independently represent a hydrogen atom; a linear or branched C₁-C₈ alkyl ; a linear or branched C₂-C₈ alkenyl; a linear or branched C₂-C₈ alkynyl; a linear or branched fluoroalkyl; a linear or branched fluoroalkenyl; a linear or branched fluoroalkynyl; a C₃-C₇ cycloalkyl; a C₄-C₇ heterocycloalkyl; a partially or totally unsaturated or aromatic C₄-C₇ carbocycle; a partially or totally unsaturated or aromatic C₅-C₇ heterocycle; a C₁-C₈ alkyl-(C₃-C₇ cycloalkyl); a C₁-C₈ alkyl-(C₄-C₇ heterocycloalkyl); a C₁-C₈ alkyl-(partially or totally unsaturated or aromatic C₄-C₇ carbocycle); a C₁-C₈ alkyl-(partially or totally unsaturated or aromatic C₅-C₇ heterocycle);
- R⁷ and R⁸ form, with the nitrogen atom to which they are bonded, a saturated, partially or totally unsaturated 4-, 5-, 6- or 7-membered heterocycle comprising at least one N atom;
- T¹ independently represents a halogen atom; an alkyl; -(X)ₓ-C₁-C₆ alkyl; a linear or branched fluoroalkyl; a linear or branched -O-C₁-C₃ fluorooalkyl; -(X)ₓ-C₃-C₆ cycloalkyl; -(X)ₓ-C₄-C₆ heterocycle; -(X)ₓ-(CT⁶T⁷)_{y}-aryl; -(X)ₓ-(CT⁶T⁷)_{y}CN; -(X)ₓ-(CT⁶T⁷)_{y}OT⁴; -(X)ₓ-(CT⁶T⁷)_{y}ST⁴; -(X)ₓ-(CT⁶T⁷)_{y}S(O)T⁴; -(X)ₓ-(CT⁶T⁷)_{y}S(O)₂T⁴; - (X)ₓ-(CT⁶T⁷)_{y}NT⁴T⁵; -(X)ₓ-(CT⁶T⁷)_{y}C(O)T⁴; -(X)ₓ-(CT⁶T⁷)_{y}C(O)OT⁴; -(X)ₓ-(CT⁶T⁷)_{y}C(O)NT⁴T⁵; -(X)ₓ-(CT⁶T⁷)_{y}NT⁴C(O)NT⁴T⁵; -(X)ₓ-(CT⁶T⁷)_{y}NT⁴C(O)T⁵; -(X)ₓ-(CT⁶T⁷)_{y}NT⁴C(O)OT⁵; -(X)ₓ-(CT⁶T⁷)_{y}OC(O)NT⁴T⁵; -(X)ₓ-(CT⁶T⁷)_{y}S(O)₂NT⁴T⁵ or-(X)ₓ-(CT⁶T⁷)_{y}NT⁴S(O)₂T⁵;
- T² independently represents a halogen atom; a linear or branched -O-C₁-C₃ alkyl; a linear or branched C₁-C₃ fluoroalkyl; a linear or branched -O-C₁-C₃ fluoroalkyl; a linear or branched C₁-C₃ alkyl; a C₃-C₆ cycloalkyl or -CN;
- two geminal T² form with the carbon atom to which they are bonded a C₃-C₇ cycloalkyl;
- T³ independently represents a linear or branched C₁-C₂ alkyl ; a fluor atom;
- T⁴ and T⁵, identical or different, independently represent a hydrogen atom; a branched or linear C₁-C₆ alkyl; a C₃-C₆ cycloalkyl;
- T⁴, T⁵ and the nitrogen atom to which they are bonded form a C₄-C₆ heterocycloalkyl;
- T⁶ and T⁷, identical or different, independently represent a hydrogen atom, a fluorine atom or a linear or branched C₁-C₃ alkyl or a C₃-C₆ cycloalkyl;
- T⁶, T⁷ and the carbon atom to which they are bonded form a C₃-C₆ cycloalkyl;
- X independently represents an oxygen atom; a sulphur atom; NT³; S=O or S(O)₂;
- Z independently represents a single bond; a linear or branched C₂-C₈ alkyl;
- x represents 0 or 1;
- y represents 0, 1, 2 or 3;
and a racemate, enantiomer, isomer, atropoisomer or diastereoisomer or a phamaceutically acceptable salt thereof.

The invention also provides a compound of formula (2B), (3B), (4B) or (5B): 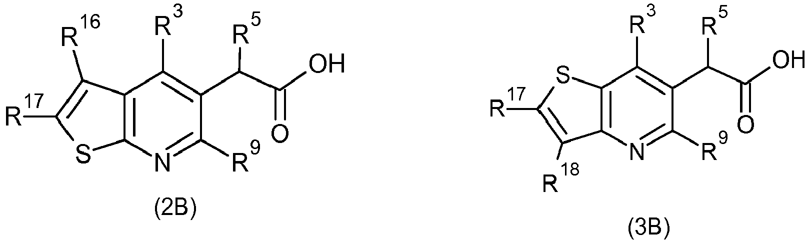 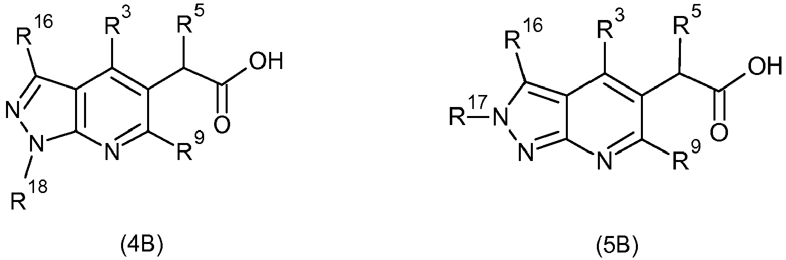 wherein R¹⁶, R¹⁷ or R¹⁸, identical or different, non-substituted or substituted by at least one T¹, independently represent a hydrogen atom, -CN, -OH, -O-cycloalkyl, -O-cycloalkenyl,-O-cycloalkynyl, -NH₂, -NR¹⁵-cycloalkyl, -NR¹⁵-cycloalkenyl, -NR¹⁵-cycloalkynyl, -S-cycloalkyl, -S-cycloalkenyl, -S-cycloalkynyl, -COOH, -C(O)NH₂, -CF₃, -SO₂NH₂, - NHSO₂NH₂, -NHC(O)NH₂, -OC(O)NH₂, halogen, alkyl, alkenyl, alkynyl, heteroalkyl, heteroalkenyl, heteroalkynyl, aryl, -O-aryl, -NR¹⁵-aryl, -S-aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, -O-heterocycle, -NR¹⁵-heterocycle, -S-heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl ;
wherein a carbon atom or a heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl can be oxidized to form a C=O, C=S, N=O, N=S, S=O or S(O)₂;
wherein the aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, or heterocyclyl-heteroalkynyl group can be fused with at least one further cycle;
wherein the alkyl, alkenyl, alkynyl, arylalkyl, arylalkenyl, arylalkynyl, cycloalkyl, cycloalkenyl, cycloalkynyl, heterocyclyl-alkyl, heterocyclyl-alkenyl, heterocyclyl-alkynyl group can include one or more heteroatoms, selected from O, S and N, in the alkyl, alkenyl, alkynyl moiety,
and R³, R⁵, R⁹, T¹, T², T³, T⁴, T⁵, T⁶, T⁷, T⁸, T⁹, T¹⁰ and T¹¹ are defined as for the compound of formula (B).

The invention also provides a compound of formula (6B), (7B), (8B), (9B): 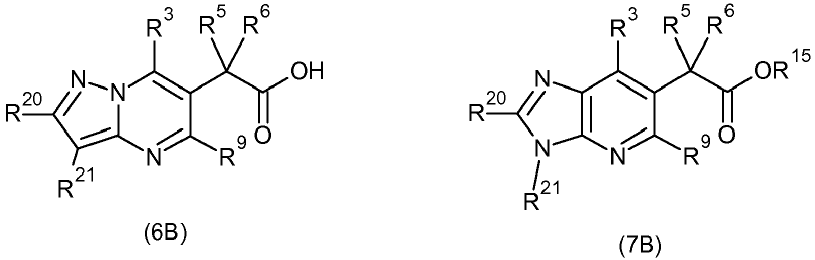 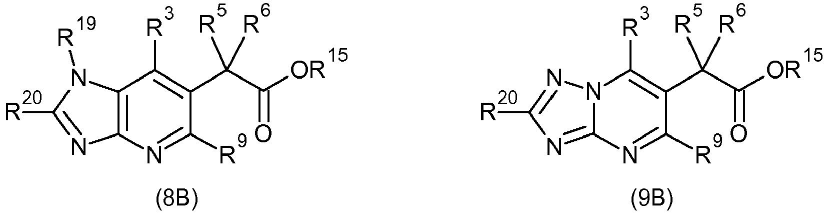 wherein:
R¹⁹, R²⁰ and R²¹, identical or different, , non-substituted or substituted by at least one T¹, independently represent a hydrogen atom, -CN, -OH, -O-cycloalkyl, -O-cycloalkenyl, -O-cycloalkynyl, -NH₂, -NR¹⁵-cycloalkyl, -NR¹⁵-cycloalkenyl, -NR¹⁵-cycloalkynyl, -S-cycloalkyl, -S-cycloalkenyl, -S-cycloalkynyl, -COOH, -C(O)NH₂,-CF₃, -SO₂NH₂, -NHSO₂NH₂, -NHC(O)NH₂, -OC(O)NH₂, halogen, alkyl, alkenyl, alkynyl, heteroalkyl, heteroalkenyl, heteroalkynyl, aryl, -O-aryl, -NR¹⁵-aryl, -S-aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, -O-heterocycle, -NR¹⁵-heterocycle, -S-heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl; wherein a carbon atom or a heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl can be oxidized to form a C=O, C=S, N=O, N=S, S=O or S(O)₂;
   wherein the aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, or heterocyclyl-heteroalkynyl group can be fused with at least one further cycle;
   wherein the alkyl, alkenyl, alkynyl, arylalkyl, arylalkenyl, arylalkynyl, cycloalkyl, cycloalkenyl, cycloalkynyl, heterocyclyl-alkyl, heterocyclyl-alkenyl, heterocyclyl-alkynyl group can include one or more heteroatoms, selected from O, S and N, in
   the alkyl, alkenyl, alkynyl moiety;
and R³, R⁵, R⁶, R⁹, R¹⁵, T¹, T², T³, T⁴, T⁵, T⁶, T⁷, T⁸, T⁹, T¹⁰ and T¹¹ are defined as for the compound of formula (B).

The invention also provides a compound of formula (10B), (11 B), (12B), (13B): 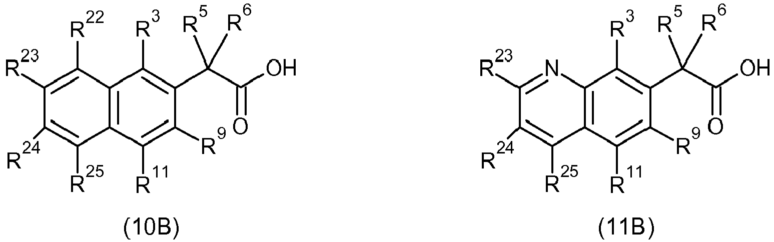 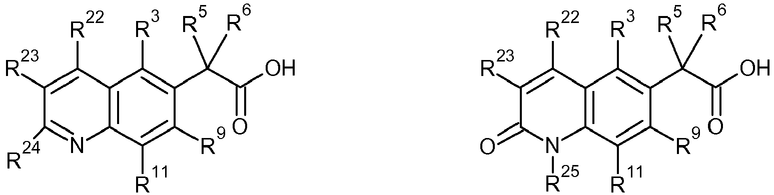 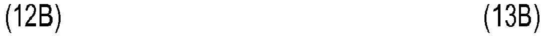 wherein:
R²², R²³, R²⁴ and R²⁵, identical or different, , non-substituted or substituted by at least one T¹, independently represent a hydrogen atom, -CN, -OH, -O-cycloalkyl,-O-cycloalkenyl, -O-cycloalkynyl, -NH₂, -NR¹⁵-cycloalkyl, -NR¹⁵-cycloalkenyl, -NR¹⁵-cycloalkynyl, -S-cycloalkyl, -S-cycloalkenyl, -S-cycloalkynyl, -COOH, -C(O)NH₂,-CF₃, -SO₂NH₂, -NHSO₂NH₂, -NHC(O)NH₂, -OC(O)NH₂, halogen, alkyl, alkenyl, alkynyl, heteroalkyl, heteroalkenyl, heteroalkynyl, aryl, -O-aryl, -NR¹⁵-aryl, -S-aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, -O-heterocycle, -NR¹⁵-heterocycle, -S-heterocycle, heterocyclyl-alkyl, heterocyclyl-alkenyl, heterocyclyl-alkynyl, heterocyclyl-heteroalkyl, heterocyclyl-heteroalkenyl,
heterocyclyl-heteroalkynyl; wherein a carbon atom or a heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl,
heterocyclyl-heteroalkynyl can be oxidized to form a C=O, C=S, N=O, N=S, S=O or S(O)₂;
wherein the aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, or heterocyclyl-heteroalkynyl group can be fused with at least one further cycle;
wherein the alkyl, alkenyl, alkynyl, arylalkyl, arylalkenyl, arylalkynyl, cycloalkyl, cycloalkenyl, cycloalkynyl, heterocyclyl-alkyl, heterocyclyl-alkenyl, heterocyclyl-alkynyl group can include one or more heteroatoms, selected from O, S and N, in the alkyl, alkenyl, alkynyl moiety;
and R³, R⁵, R⁶, R⁹, R¹¹, R¹⁵, T¹, T², T³, T⁴, T⁵, T⁶, T⁷, T⁸, T⁹, T¹⁰ and T¹¹ are defined as for the compound of formula (B).

The invention also provides a compound of formula (14B), (15B), (16B): 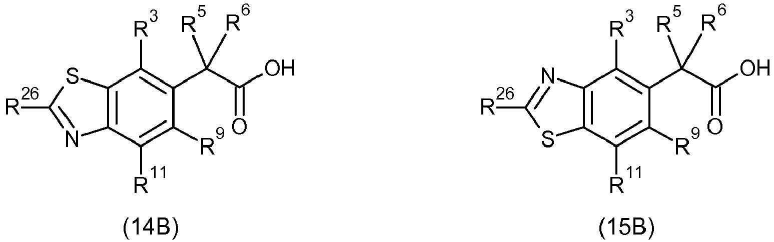 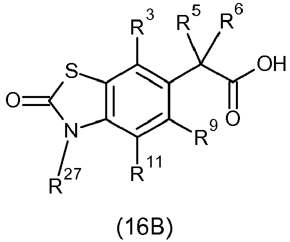 wherein:
- R²⁶ and R²⁷, identical or different, , non-substituted or substituted by at least one T¹, independently represent a hydrogen atom, -CN, -OH, -O-cycloalkyl, -O-cycloalkenyl, -O-cycloalkynyl, -NH₂, -NR¹⁵-cycloalkyl, -NR¹⁵-cycloalkenyl, -NR¹⁵-cycloalkynyl, -S-cycloalkyl, -S-cycloalkenyl, -S-cycloalkynyl, -COOH, -C(O)NH₂,-CF₃, -SO₂NH₂, -NHSO₂NH₂, -NHC(O)NH₂, -OC(O)NH₂, halogen, alkyl, alkenyl, alkynyl, heteroalkyl, heteroalkenyl, heteroalkynyl, aryl, -O-aryl, -NR¹⁵-aryl, -S-aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, -O-heterocycle, -NR¹⁵-heterocycle, -S-heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl;
   wherein a carbon atom or a heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl can be oxidized to form a C=O, C=S, N=O, N=S, S=O or S(O)₂;
   wherein the aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, or heterocyclyl-heteroalkynyl group can be fused with at least one further cycle;
   wherein the alkyl, alkenyl, alkynyl, arylalkyl, arylalkenyl, arylalkynyl, cycloalkyl, cycloalkenyl, cycloalkynyl, heterocyclyl-alkyl, heterocyclyl-alkenyl, heterocyclyl-alkynyl group can include one or more heteroatoms, selected from O, S and N, in
   the alkyl, alkenyl, alkynyl moiety;
and R³, R⁵, R⁶, R⁹, R¹¹, R¹⁵, T¹, T², T³, T⁴, T⁵, T⁶, T⁷, T⁸, T⁹, T¹⁰ and T¹¹ are defined as for the compound of formula (B).

The invention also provides a compound of formula (17B), (18B), (19B): 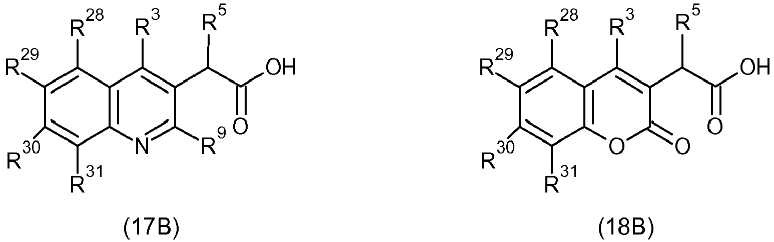 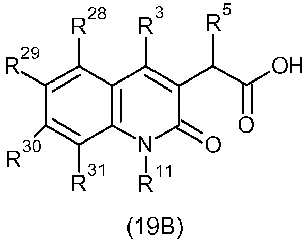 wherein:
- R²⁸, R²⁹, R³⁰ and R³¹, identical or different, , non-substituted or substituted by at least one T¹, independently represent a hydrogen atom, -CN, -OH, -O-cycloalkyl,-O-cycloalkenyl, -O-cycloalkynyl, -NH₂, -NR¹⁵-cycloalkyl, -NR¹⁵-cycloalkenyl, -NR¹⁵-cycloalkynyl, -S-cycloalkyl, -S-cycloalkenyl, -S-cycloalkynyl, -COOH, -C(O)NH₂,-CF₃, -SO₂NH₂, -NHSO₂NH₂, -NHC(O)NH₂, -OC(O)NH₂, halogen, alkyl, alkenyl, alkynyl, heteroalkyl, heteroalkenyl, heteroalkynyl, aryl, -O-aryl, -NR¹⁵-aryl, -S-aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, -O-heterocycle, -NR¹⁵-heterocycle, -S-heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl;
   wherein a carbon atom or a heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl can be oxidized to form a C=O, C=S, N=O, N=S, S=O or S(O)₂;
   wherein the aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, or heterocyclyl-heteroalkynyl group can be fused with at least one further cycle;
   wherein the alkyl, alkenyl, alkynyl, arylalkyl, arylalkenyl, arylalkynyl, cycloalkyl, cycloalkenyl, cycloalkynyl, heterocyclyl-alkyl, heterocyclyl-alkenyl, heterocyclyl-alkynyl group can include one or more heteroatoms, selected from O, S and N, in
   the alkyl, alkenyl, alkynyl moiety;
and R³, R⁵, R⁹, R¹¹, R¹⁵, T¹, T², T³, T⁴, T⁵, T⁶, T⁷, T⁸, T⁹, T¹⁰ and T¹¹ are defined as for the compound of formula (B).

The invention also provides a compound of formula (20B), (21 B) or (22B): 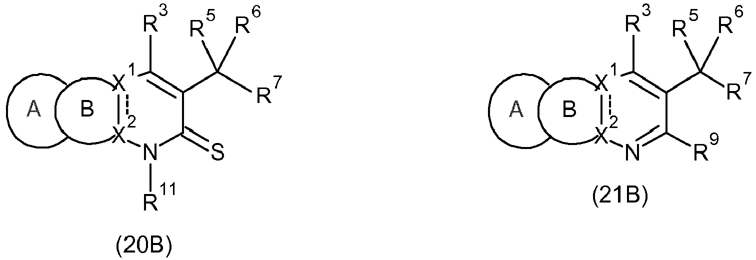 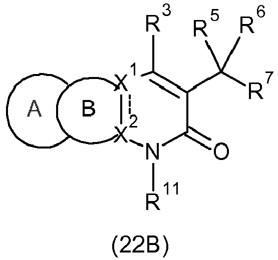 wherein,
- X¹, X², A and B form a saturated, partially or totally unsaturated 10-, 11-, 12-, 13- or 14-membered polycarbo- or polyheterocycle,
- X¹ and X², independently represent C or N,
   wherein a carbon atom or heteroatom of said alkyl, alkenyl, alkynyl, aryl, heterocycle, arylalkyl, arylalkenyl, arylalkynyl, heterocyclyl-alkyl, heterocyclyl-alkenyl, heterocyclyl-alkynyl group can be oxidized to form a C=O, C=S, N=O, N=S, S=O or S(O)₂.
   and R³, R⁵, R⁶, R⁷, R⁹, R¹¹, R¹⁵, T¹, T², T³, T⁴, T⁵, T⁶, T⁷, T⁸, T⁹, T¹⁰ and T¹¹ are defined as for the compound of formula (B).

The invention also provides a compound of formula (23B): 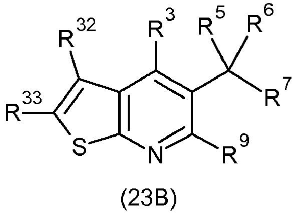 wherein,
- R³² and R³³ form together a 4-, 5-, 6-, 7- or 8-membered partially or totally unsaturated or aromatic carbocycle, a 4-, 5-, 6-, 7- or 8-membered partially or totally unsaturated or aromatic heterocycle, non-substituted or substituted by at least one T¹.
   wherein said 4-, 5-, 6-, 7- or 8-membered partially or totally unsaturated or aromatic carbocycle, a 4-, 5-, 6-, 7- or 8-membered partially or totally unsaturated or aromatic heterocycle can be oxidized to form a C=O, C=S, N=O, N=S, S=O or S(O)₂.
and R³, R⁵, R⁶, R⁷, R⁹, R¹⁵, T¹, T², T³, T⁴, T⁵, T⁶, T⁷, T⁸, T⁹, T¹⁰ and T¹¹ are defined as for the compound of formula (B).

The invention also provides a compound of formula (24B), (25B), (26B) or (27B): 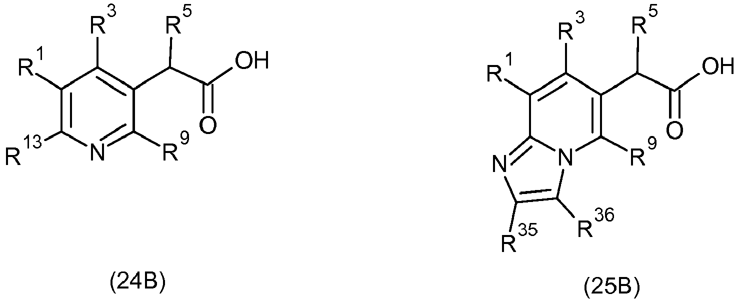 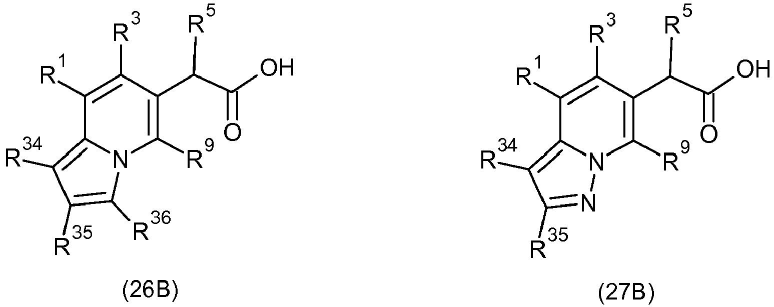 wherein:
R³⁴, R³⁵ and R³⁶, identical or different, , non-substituted or substituted by at least one T¹, independently represent a hydrogen atom, -CN, -OH, -O-cycloalkyl, -O-cycloalkenyl, -O-cycloalkynyl, -NH₂, -NR¹⁵-cycloalkyl, -NR¹⁵-cycloalkenyl, -NR¹⁵-cycloalkynyl, -S-cycloalkyl, -S-cycloalkenyl, -S-cycloalkynyl, -COOH, -C(O)NH₂,-CF₃, -SO₂NH₂, -NHSO₂NH₂, -NHC(O)NH₂, -OC(O)NH₂, halogen, alkyl, alkenyl, alkynyl, heteroalkyl, heteroalkenyl, heteroalkynyl, aryl, -O-aryl, -NR¹⁵-aryl, -S-aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, -O-heterocycle, -NR¹⁵-heterocycle, -S-heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl; wherein a carbon atom or a heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl can be oxidized to form a C=O, C=S, N=O, N=S, S=O or S(O)₂;
   wherein the aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, or heterocyclyl-heteroalkynyl group can be fused with at least one further cycle;
   wherein the alkyl, alkenyl, alkynyl, arylalkyl, arylalkenyl, arylalkynyl, cycloalkyl, cycloalkenyl, cycloalkynyl, heterocyclyl-alkyl, heterocyclyl-alkenyl, heterocyclyl-alkynyl group can include one or more heteroatoms, selected from O, S and N, in the alkyl, alkenyl, alkynyl moiety;
and R³, R⁵, R⁹, R¹³, R¹⁵, T¹, T², T³, T⁴, T⁵, T⁶, T⁷, T⁸, T⁹, T¹⁰ and T¹¹ are defined as for the compound of formula (B).

The invention also provides a compound of formula (28B): 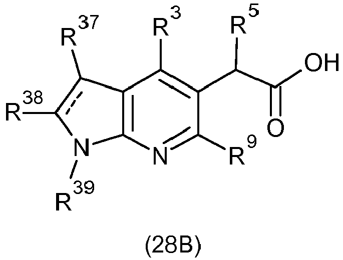 wherein:
R³⁷, R³⁸ and R³⁹, identical or different, , non-substituted or substituted by at least one T¹, independently represent a hydrogen atom, -CN, -OH, -O-cycloalkyl, -O-cycloalkenyl, -O-cycloalkynyl, -NH₂, -NR¹⁵-cycloalkyl, -NR¹⁵-cycloalkenyl, -NR¹⁵-cycloalkynyl, -S-cycloalkyl, -S-cycloalkenyl, -S-cycloalkynyl, -COOH, -C(O)NH₂,-CF₃, -SO₂NH₂, -NHSO₂NH₂, -NHC(O)NH₂, -OC(O)NH₂, halogen, alkyl, alkenyl, alkynyl, heteroalkyl, heteroalkenyl, heteroalkynyl, aryl, -O-aryl, -NR¹⁵-aryl, -S-aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, -O-heterocycle, -NR¹⁵-heterocycle, -S-heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl.wherein a carbon atom or a heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl can be oxidized to form a C=O, C=S, N=O, N=S, S=O or S(O)₂;
   wherein the aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, or heterocyclyl-heteroalkynyl group can be fused with at least one further cycle;
   wherein the alkyl, alkenyl, alkynyl, arylalkyl, arylalkenyl, arylalkynyl, cycloalkyl, cycloalkenyl, cycloalkynyl, heterocyclyl-alkyl, heterocyclyl-alkenyl, heterocyclyl-alkynyl group can include one or more heteroatoms, selected from O, S and N, in the alkyl, alkenyl, alkynyl moiety;
• R³⁷ and R³⁸ form together a (C₄-C₇)cycloalkyl, a (C₃-C₉)heterocycle, a (C₅-C₁₄)aryl,
• R³⁸ and R³⁹ form together a (C₃-C₇)heterocycle;
and R³, R⁵, R⁹, R¹⁵, T¹, T², T³, T⁴, T⁵, T⁶, T⁷, T⁸, T⁹, T¹⁰ and T¹¹ are defined as for the compound of formula (B).
The invention also provides a compound of formula (29B): 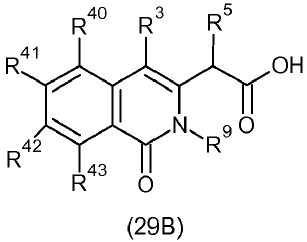 wherein:
R⁴⁰, R⁴¹ R⁴² and R⁴³, identical or different, non-substituted or substituted by at least one T¹, independently represent a hydrogen atom, -CN, -OH, -O-cycloalkyl,-O-cycloalkenyl, -O-cycloalkynyl, -NH₂, -NR¹⁵-cycloalkyl, -NR¹⁵-cycloalkenyl, -NR¹⁵-cycloalkynyl, -S-cycloalkyl, -S-cycloalkenyl, -S-cycloalkynyl, -COOH, -C(O)NH₂,-CF₃, -SO₂NH₂, -NHSO₂NH₂, -NHC(O)NH₂, -OC(O)NH₂, halogen, alkyl, alkenyl, alkynyl, heteroalkyl, heteroalkenyl, heteroalkynyl, aryl, -O-aryl, -NR¹⁵-aryl, -S-aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, -O-heterocycle, -NR¹⁵-heterocycle, -S-heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynylwherein a carbon atom or a heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl can be oxidized to form a C=O, C=S, N=O, N=S, S=O or S(O)₂;
   wherein the aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, or heterocyclyl-heteroalkynyl group can be fused with at least one further cycle;
   wherein the alkyl, alkenyl, alkynyl, arylalkyl, arylalkenyl, arylalkynyl, cycloalkyl, cycloalkenyl, cycloalkynyl, heterocyclyl-alkyl, heterocyclyl-alkenyl, heterocyclyl-alkynyl group can include one or more heteroatoms, selected from O, S and N, in
   the alkyl, alkenyl, alkynyl moiety;
and R³, R⁵, R⁹, R¹⁵, T¹, T², T³, T⁴, T⁵, T⁶, T⁷, T⁸, T⁹, T¹⁰ and T¹¹ are defined as for the compound of formula (B).

The invention also provides a compound of formula (30B), (31 B) or (32B): 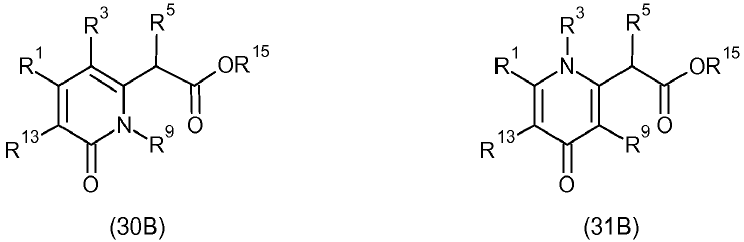 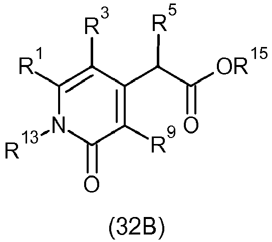 wherein :
R¹, R³, R⁵, R⁹, R¹³ , R¹⁵, T¹, T², T³, T⁴, T⁵, T⁶, T⁷, T⁸, T⁹, T¹⁰ and T¹¹ are defined as for the compound of formula (B).

The invention also provides a compound of formula (33B): 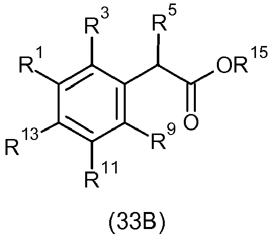 whereinR¹ R³, R⁵, R⁹, R¹¹ R¹³, R¹⁵, T¹ T², T³, T⁴, T⁵, T⁶, T⁷, T⁸, T⁹, T¹⁰ and T¹¹ are defined as for the compound of formula (B).

The invention also provides a compound of formula (34B), (35B) or (36B): 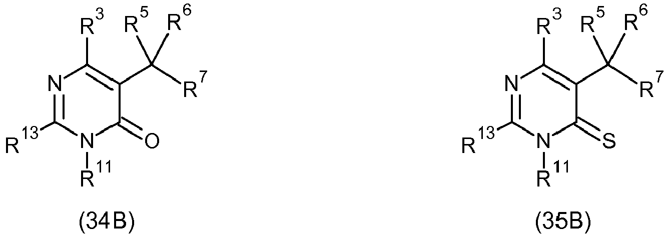 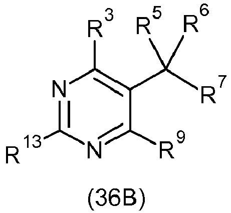 wherein R³, R⁵, R⁶, R⁹, R¹¹, R¹³, R¹⁵, T¹, T², T³ , T⁴, T⁵, T⁶, T⁷, T⁸, T⁹, T¹⁰ and T¹¹ are defined as for the compound of formula (B).

The present invention will now be described in further detail using examples that have to be taken as non-limiting embodiments.

### Description of the Figures:

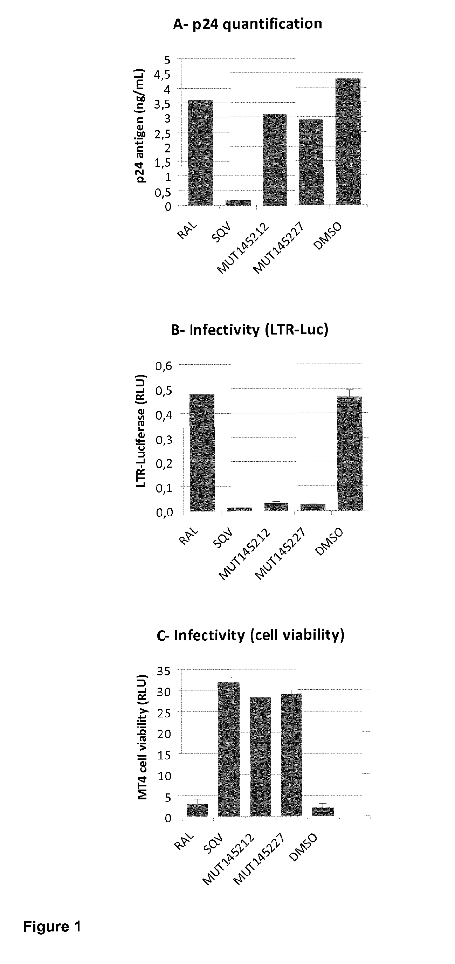

Figure 1. Analysis of the production and the infectivity of LAV virus particles produced by Hela-LAV cells treated with the indicated compounds. (A) Titration of p24 harvested from HeLa-LAV cells treated with the indicated compounds. **(B)** Infectivity of virions harvested from HeLa-LAV cells treated with the indicated compounds and tested by infection of TZM indicator cells and luciferase assay. **(C)** Infectivity of virions harvested from Hela-LAV cells treated with the indicated compounds and tested by infection of MT4 cells and cytopathic assay using CellTiter-Glo®.

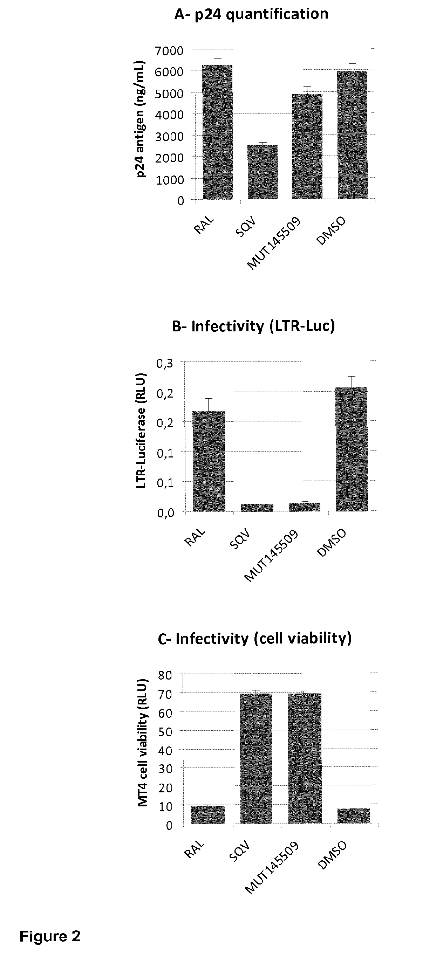

**Figure 2****.** Analysis of the production and the infectivity of LAV virus particles produced by Hela-LAV cells treated with the indicated compounds. **(A)** Titration of p24 harvested from HeLa-LAV cells treated with the indicated compounds. **(B)** Infectivity of virions harvested from HeLa-LAV cells treated with the indicated compounds and tested by infection of TZM indicator cells and luciferase assay. **(C)** Infectivity of virions harvested from Hela-LAV cells treated with the indicated compounds and tested by infection of MT4 cells and cytopathic assay using CellTiter-Glo®.

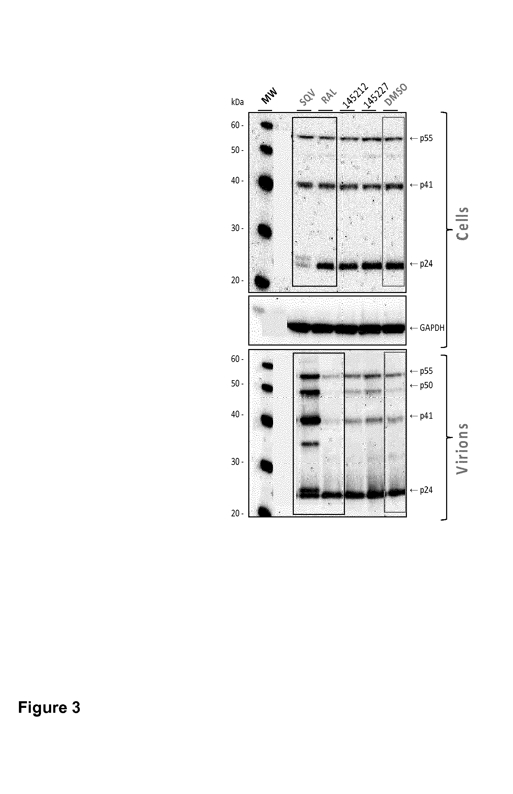

**Figure 3****:** Western blot analysis of Gag maturation in HIV-1 NL4-3 producer cells (upper panel) and in the content of Gag proteins in virions (lower panel) after treatment of producer cells with the indicated compounds, using p24 antibody

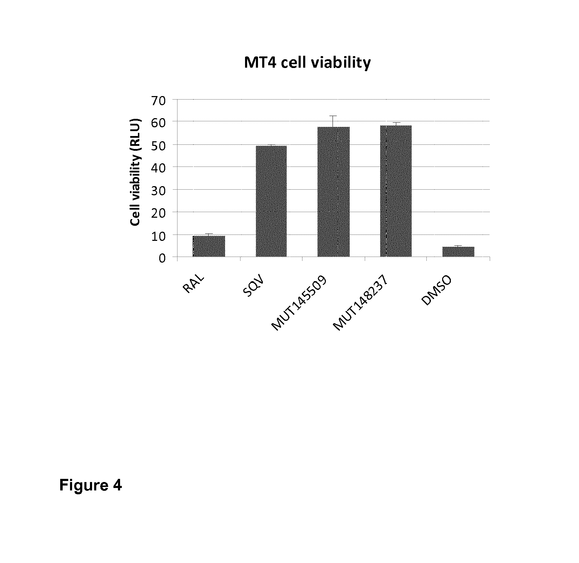

**Figure 4****:** Infectivity of wt NL4-3 viruses harvested from 293T transfected cells after treatment with the indicated compounds and tested by infection of MT4 cells using the cytopathic assay CellTiter-Glo®.
**Figure 5:** Multimerization of HIV-1 IN upon treatment with inactivating compounds Mut145212 and 145240. In the absence of incubation with Mut145212 or Mut145240, HIV-1 Integrase (IN) is eluted as a protein with a MW corresponding to a dimer of Integrase of 64 KD (upper panels peaks in red). After incubation with Mut145212 or Mut145240 this peak of IN dimer is displaced and eluted earlier with a MW corresponding to an IN tetramer of 130 KD (upper panels peaks in blue). In contrast, incubation with raltegravir (Merck) (lower panel) has no effect on IN (peak in red) which is eluted as an IN dimer as the untreated IN (peak in blue).

### Examples:

### Example 1: Structure of IN-LEDGF allosteric inhibitor compounds that can be used to inactivate HIV in order to use the inactivated viruses in vaccine preparations:

IN-LEDGF allosteric inhibitors (INLAIs) of the aryl or heteroaryl-tertbutoxy-acetic acid family described in WO2012/140243, WO2012/137181 and Le Rouzic et al. (abstract #547 CROI conference March 3-6, 2013, Atlanta, USA), all compounds that can bind to the LEDGF-binding pocket of HIV-1 integrase and promote inactivation of HIV-1 when treating HIV producer cells during virus production can be used to inactivate HIV, such as compounds listed on table 1: Mut145184 was synthesized as racemic compound according example BI-D described in Fenwick et al. CROI 2011 and compound 10006 in WO2009/062285. Mut145212, Mut145227 and Mut145240 (which are compounds 1039, 3014 and 1078 respectively in WO2009/062289) were synthesized as described in WO2009/062289. Mut145249, Mut145347, Mut145362, Mut145375, Mut145429, Mut145509, Mut145535 were synthesized as described in examples 2, 15, 17, 18, 20, 26, 29 respectively, in WO2012/140243. Mut145871 was prepared using the method described for example 4 in WO2012/3497 Mut148237 was prepared using the method described in EP Application no. 12187528.0.

The structure and activities of these compounds are shown on Table 1.

**Table 1: Structure and activity of IN-LEDGF inhibitors designed in this study**

| **Structure** | **Compound** | **MW (g/mol)** | **Biochemical assays** | | | **MT4 assays** | | **Reference** |
|---|---|---|---|---|---|---|---|---|
| | | | **CCD-IBD IC50** | **IN-LEDGF IC50** | **IN-IN IC50** | **NL4.3 EC50** | **HxB2 EC50** | |
| 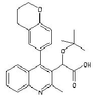 | MUT145184= racemic BI-D | 405 | +++ | +++ | +++ | +++ | +++ | WO2009/06228 5 |
| 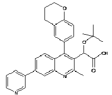 | MUT145212 | 483 | +++ | +++ | +++ | +++ | +++ | WO2009/06228 9 |
| 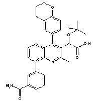 | MUT145227 | 525 | +++ | +++ | +++ | +++ | +++ | WO2009/06228 9 |
| 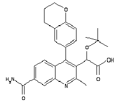 | MUT145240 | 449 | +++ | +++ | +++ | ++ | +++ | WO2009/06228 9 |
| 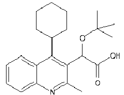 | MUT145249 | 355 | + | ++ | +++ | ++ | +++ | WO2012/14024 3 |
| 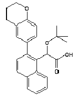 | MUT145347 | 390 | ++ | ++ | +++ | + | + | WO2012/14024 3 |
| 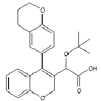 | MUT145362 | 394 | ++ | ++ | +++ | ++ | ++ | WO2012/14024 3 |
| 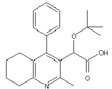 | MUT145375 | 353 | + | ++ | ++ | ++ | ++ | WO2012/14024 3 |
| 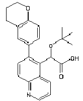 | MUT145429 | 391 | ++ | ++ | +++ | ++ | ++ | WO2012/14024 3 |
| 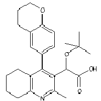 | Mut101 = MUT145509 | 410 | +++ | +++ | +++ | +++ | +++ | WO2012/14024 3 |
| 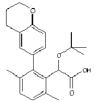 | MUT145535 | 368 | ++ | +++ | +++ | ++ | +++ | WO2012/14024 3 |
| 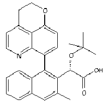 | MUT145871 | 442 | +++ | +++ | +++ | +++ | +++ | WO2012/00349 7 |
| 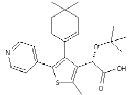 | MUT148237 | 414 | +++ | +++ | +++ | +++ | +++ | EP 12187528.0 |

Structure, molecular weight, biochemical activity on IN-LEDGF or IN/CCD-LEDGF/IBD interaction inhibition, IN-IN interaction enhancement, and antiretroviral activities on HIV-1 NL4-3 and HXB2 virus isolates, of compounds, are listed. NT or ND = not tested. IC₅₀ = concentration required to inhibit IN-LEDGF or IN/CCD-LEDGF/IBD interaction by 50%; AC₅₀= concentration required to activate IN-IN interaction by 50%; EC₅₀ = concentration required to inhibit HIV-1 infection of MT4 cells by 50%.
+ means an activity comprises between 20 and 10 micromolar concentration
++ means an activity comprises between 10 and 1 micromolar concentration
+++ means an activity below 1 micromolar concentration.

As indicated on table 1, these compounds efficiently inhibited IN-CCD/LEDGF-IBD interaction as well as interaction between IN and LEDGF full length proteins in Homogeneous Time Resolved Fluorescence (HTRF) assays. Also these compounds efficiently enhance IN-IN interaction in HTRF assay, this result being in favor of a multimerization of IN promoted by the binding of active compounds to the LEDGF binding pocket of IN. We found a good correlation between their ARV activity studied by infection of MT4 cells with HxB2 HIV-1 and their ability to inhibit IN-CCD/LEDGF-IBD or IN-LEDGF or to enhance IN-IN interactions.

Compounds Mut145184, Mut145212, Mut145347, Mut145362, Mut145509, Mut145871, Mut148237 have been co-crystallized with the IN-CCD dimer, showing that their binding pocket on IN corresponds indeed to the LEDGF-binding site.

As an example, two molecules of Mut145509 are bound to the IN-CCD dimer. Mut145509 is in a pocket surrounded by hydrophobic residues on one side, acidic region on the other side and basic residues in the bottom of the pocket. Three hydrogen bonds are made between the carboxylic acid group of Mut145509 and the protein, one with the hydroxyl group of the side chain of Thr 174, and two with the amino group of the main chain of His171 and Glu170. In addition Mut145509 interacts with two water molecules (Le Rouzic et al. abstract #547 CROI conference March 3-6, 2013, Atlanta, USA).

All compounds that can bind to the LEDGF-binding pocket of HIV-1 integrase and promote inactivation of HIV-1 when treating HIV producer cells during virus production can also be used to inactivate HIV, such as compounds described in:
- WO2007/131350
- WO2008/067644,
- WO2009/062285,
- WO2009/062288
- WO2009/062289
- WO2009/062308
- WO2010/130034
- Fenwick, C. et al., Identification of BI-C, a novel HIV-1 non-catalytic site integrase inhibitor, in 18th CROI2011: Boston,
- WO2010/130842,
- WO2011/015641,
- WO2011/076765,
- WO2012/140243,
- WO2012/137181
- WO2012/003497,
- WO2012/003498,
- WO2012/066442,
- WO2012/033735,
- WO2012/102985,
- WO2012/145728,
- WO2012/138669
- WO2012/065963
- EP Application No. 12187528.0
- WO2013/002357,
- WO2013/043553
- US2013/0018049
- WO2013/062028
- WO2013/025584
- Christ F et al., Antimicrob Agents Chemother. 2012, 56(8):4365-74
- Tsiang M et al., J Biol Chem. 2012, 287(25):21189-203.
- WO 2013/073875

Since the binding site for IN-LEDGF inhibitors lies at the IN dimer interface, we evaluated the ability of these inhibitors to promote modifications in the interaction between IN subunits. We designed an HTRF-based assay to monitor interaction between His₆-IN/Flag-IN subunits. In the presence of increasing compound concentrations the HTRF signal corresponding to the His₆-IN/Flag-IN interaction was increased by more than two fold (200%) compared to the signal obtained in the absence of compound. The concentration required to activate IN-IN interaction by 50% (AC₅₀) closely correlated with the inhibition of IN-LEDGF interaction (Table 1). By contrast, Raltegravir had no effect, neither on IN-LEDGF interaction nor on IN-IN interaction (data not shown). These results are in agreement with previously reported observations on the effect of some LEDGINs and tBPQAs on IN-IN interaction (Christ et al., 2012; Kessl et al., 2012; Tsiang et al., 2012), and confirm that IN-LEDGF inhibitors, besides their ability to inhibit IN-LEDGF interaction are true allosteric inhibitors of IN that promote conformational change of IN by binding to the LEDGF-binding pocket, mimicking the effect of LEDGF binding to IN (Hare et al., 2009 PLoS Pathog 5, e1000515.; Hayouka et al., 2007 Proc Natl Acad Sci U S A 104, 8316-8321.).

### Example 2: Inactivation of HIV infectious viruses by treating Hela-LAV cell line as HIV-LAV producer cell with compounds:

In order to prove that compounds are able to inactivate HIV during virus production, we used the Hela-LAV system in which the Hela cell line has been transduced by HIV-1 LAV virus (Berg J et al., J. Virol. Methods. 1991 Sep-Oct; 34(2):173-80). In this cell line HIV-1 LAV is constitutively integrated and Hela-LAV cells produce HIV-1 LAV virions that cannot re-infect these cells since they do not express CD4 at their surface. Therefore, in this cell line only drugs that act during virus production, at late steps post-integration of the HIV-1 replication cycle, are expected to be able to inactivate HIV during virus production.

Hela-LAV cells were treated with inactivating antiretroviral compounds such as Mut145212, Mut145227, Mut145509, or reference antiretroviral drugs like Raltegravir (Merck) that are not active at production stage, or Protease inhibitors such as Saquinavir (SQV) that are able to inactivate HIV at production stage or DMSO as negative control. In order to examine the infectivity of viruses produced in the presence of these various compounds, the supernatants were harvested, titrated for viral protein p24 release using the Alliance HIV-1 p24 Antigen ELISA (PerkinElmer, http://www.perkinelmer.com/) and titrated to measure the quantity of infectious particles per ml by infecting TZM-bl indicator cells (from the AIDS reagent program, NIH) expressing luciferase under a Tat-dependent promoter. Alternatively for titration of infectious particles, target cells for HIV-1 infection such as MT4 cells were used.

Viruses harvested were first titrated by p24 assay, showing that the amounts of p24 produced in the presence of compounds Mut145212, Mut145227, and Mut145509 were comparable to that in the presence of DMSO, Raltegravir (RAL) (Merck), (Figure 1A &2A). In contrast, as expected, a much lower amount of p24 (30%) was produced after treatment by the protease inhibitor SQV (Figure 1A & 2A). Infectivity of viruses produced in the presence of Raltegravir was comparable to viruses produced in the presence of DMSO, as measured on TZM indicator cells by luciferase assays (Figure 1B & 2B). In contrast, viruses produced during Mut145212, Mut145227 or Mut145509 treatment were non-infectious in comparison to fully infectious viruses produced in the presence of Raltegravir or DMSO ((Figure 1B & 2B).. The absence of infectivity of viruses produced in the presence of Mut145212, Mut145227 or Mut145509 was in the same order than non infectious viruses produced after SQV treatment (Figure 1B & 2B). This loss in infectivity resulting from Mut145212, Mut145227 or Mut145509 treatment was confirmed by infection of MT4 cells titrated by cytopathic effect using CellTiter-Glo® assay (Figure 1C & 2C).. As shown in (Figure 1C & 2C), viruses produced in the presence of Raltegravir are fully infectious and provoke a cytopathic effect on MT4 cells comparable to infection with viruses harvested after treatment with DMSO showing that Raltegravir treatment during virus production did not alter infectivity. In contrast, viruses produced in the presence of Mut145212, Mut145227, or Mut145509, similarly to those produced in the presence of SQV, were totally impaired for such cytopathic effect, confirming the absence of infectivity detected on TZM cells (Figure 1C & 2C). This infectivity defect cannot be due to residual concentration of Mut145212, Mut145227 or Mut145509 used during virus production since the virus stock was diluted up to 2000 times before infection, lowering compound concentration much below their effective EC₅₀ concentration. By western blot using anti-p24 antibody, we could not detect any perturbation of Gag maturation and CA p24 content, by analysis of HeLa-LAV cell lysates and defective virions produced in the presence of Mut145212, Mut145227 (Figure 3), neither in the presence of Mut145509.

### Example 3:

### Inactivation of HIV infectious viruses by treatment, with the ARV compounds Mut145509 and Mut148237, of 293T producer cells transfected with HIV molecular clones.

HIV-1 NL4-3 virus was produced upon 293T cell transfection in the presence of Mut145509, Mut148237, SQV or DMSO. 2 hours after transfection indicated compounds were added during virus production for 48 hours at the indicated concentrations. Then supernatants were diluted 2000 times to decrease compound concentration much lower than their respective EC₅₀. Viruses released in cell supernatants were harvested and tested for virus production by p24 assay, and virus infectivity by infection of MT4 cells and cytophatic assay using CellTiter-Glo® (Promega) according manufacturer's instructions.

As shown in Figure 4, NL4-3 virus produced in the presence of Mut145509, Mut148237, or Saquinavir (SQV) used as Protease inhibitor control, was inactivated by such treatments and viability of MT4 cells infected by these viruses was preserved, in contrast, viruses treated with DMSO retained full infectivity that resulted in MT4 cell death. Raltegravir (Merck) treatment during virus production had no effect on viruses that conserved full infectivity comparable to that observed with DMSO, an inactive analog of Mut145509 and Mut148237.

### Example 4: Multimerization of recombinant HIV-1 Integrase upon treatment with inactivating compounds

Multimerization of HIV-1 Integrase upon treatment with inactivating compounds was performed using size exclusion chromatography on a Superdex 200 PC 3.2/30 column (GE Healthcare), as described in the method section. Aldolase (158,000 MW), Conalbumin (75,000 MW), Carbonic Anhydrase (29,000 MW), and Ribonuclease A (13,700 MW) were used as protein markers for calibration. As shown in Figures 5, in the absence of incubation with inactivating compounds, HIV-1 integrase (IN) is eluted at 1.74ml as a protein corresponding quite well to the expected elution of a MW of an IN dimer (64KD MW). After incubation with inactivating compounds Mut145212 or Mut 145240 (Figure 5 upper panels), HIV-1 Integrase (IN) was displaced and eluted at 1.55ml as an IN tetramer (130 KD MW). Treatment of IN with raltegravir (Merck) (Figure 5 lower panel) has no effect on IN which is eluted as an IN dimer as the untreated IN. These results are in complete agreement with the enhancement of IN-IN interaction found upon treatment with inactivating compounds using HTRF assay (see table 1). These results demonstrate also that this multimerization of IN is specific for treatment with inactivating compounds and is not found with other ligands of IN such as raltegravir that binds to another binding site on IN which is the catalytic site, which is different from the binding site of inactivating compounds which is the LEDGF-binding pocket on IN.

### Methods:

**Compounds:** Control compounds such as Saquinavir (SQV), Indinavir (IDV), Nevirapine (NVP), Efavirenz (EFV) and AZT were obtained from the NIH AIDS research and Reference Reagent Program. Raltegravir (RAL) (Merck) and Elvitegravir (EVG) (Gilead) were purchased from Selleck Chemicals (Munich, Germany).

**Cell culture:** MT-4, TZM-bl and HeLa-LAV cells were obtained through the AIDS Research and Reference Reagent Program, Division of AIDS, NIAID, NIH. MT-4 cells were grown in RPMI 1640 supplemented with 10% heat-inactivated fetal calf serum and 100 IU/ml penicillin, and 100 µg/ml streptomycin (Invitrogen) to obtain RPMI-complete. HeLa-LAV, TZM-bl and 293T cells (ATCC, CRL-11268) were grown in DMEM supplemented with 10% FCS and antibiotics. TZM-bl cells are a HeLa modified cell line containing separately integrated copies of the luciferase and β-galactosidase genes under control of the HIV-1 promoter.

**Virus strains and recombinant HIV-1 molecular clones:** HIV-1 NL4-3 and HXB2 molecular clones sequences are in (Stanford University HIV Drug Resistance Database).

**Viral stock:** 293T (2 × 10⁶ cells) were transfected with 6 µg of pNL4-3 proviral plasmids (wild-type or drug resistant) using X-tremeGENE 9 reagent (Roche). Cell were washed 24h later and cell supernatants were collected 48 h post-transfection and stored at -80°C. All Viral stocks were quantified for p24 antigen using the Alliance HIV-1 p24 Antigen ELISA (PerkinElmer, http://www.perkinelmer.com/) and titrated to measure the quantity of infectious particles per ml by infecting TZM-bl indicator cells.

**Antiviral assay in MT-4 cells:** MT-4 cells (ATCC) growing exponentially at the density of 10⁶/ml were infected with HIV-1 strain NL4-3 at a MOI of 0.00001 during two hours. The cells were then washed with PBS and then aliquoted in 100 µL fresh complete RPMI to 96-well white plates (Costar) in the presence of varying concentrations of compounds. The effective concentration of compound required to inhibit 50% (EC₅₀) of HIV-1 replication was determined after 5 days using the CellTiter-Glo^{®} luminescent reagent (Promega, France) to quantify cell viability.

### Constructions of epitope-tagged proteins:

His₆-LEDGF plasmid was previously described (Michel et al., 2009, EMBO J 28, 980-991.). Plasmid encoding GST-Flag-IBD/LEDGF was performed by cloning LEDGF DNA sequence encoding residues 342 to 507 in fusion with the Flag epitope into pGEX-2T (GE Healthcare). His₆-IN plasmid corresponds to pINSD.His and was previously described (Bushman et al., 1993, Proc Natl Acad Sci U S A 90, 3428-3432). Full length FLAG-tagged integrase sequence from NL4-3 was PCR amplified and cloned between BamHI and Xhol restriction sites of pGEX-6P1 vector (GE Healthcare) to generate the expression plasmid GST-Flag-IN. His-CCD and GST-Flag-CCD were obtained by cloning the integrase region encoding the catalytic core domain (residues 50 to 202) from pINSD.His.Sol (Jenkins et al., 1995) into pET15b and pGEX-2T-Flag, respectively. Thereby the CCD contains the F185K mutation which greatly improves the solubility of the recombinant protein. CCD T174I mutation was introduced into the His-CCD plasmid by site-directed mutagenesis.

### Purification of recombinant proteins:

Frozen cells pellets corresponding to one liter culture were re-suspended in 3.5 mL of integrase buffer (50 mM HEPES pH 7.5, 1 M NaCl, 7 mM CHAPS, 5 mM MgCl₂, 2 mM β-mercaptoethanol, 10% glycerol) for full length integrase or a 2 fold dilution in water of the same buffer for integrase CCD, containing Complete™ protease inhibitor cocktail (Roche) and benzonase (Sigma). Cells were disrupted using 25 g-30 g 150-212 µm glass beads (Sigma) and vortex at 4°C during 10 min. Glass beads were washed 3 times with 15 mL of extraction buffer and whole cell lysate was centrifuged at 109,000 g (Rₘₐₓ) for 1 h at 4°C in a Beckman XL80K ultracentrifuge.
- His₆-tagged IN or His₆-tagged IN-CCD lysate was loaded at 3 mL/min on 5 mL His-Trap FF crude column (GE Healthcare) previously equilibrated with integrase buffer or CCD buffer, respectively, containing 20 mM imidazole. After washing until OD₂₈₀ₙₘ returned to the baseline, bound proteins were eluted using a 20 to 500 mM imidazole gradient in 20 column volume. Pooled fractions were concentrated to 2.5 mL using Amicon Ultra 15™ 10 K centrifugal filter devices (Millipore) at 4,000 g at 4°C. Concentrated protein was loaded on a Superdex 200 16/600 PG column for IN full length or a Superdex 75 16/600 PG column for IN-CCD (GE Healthcare) previously equilibrated with integrase buffer at 4°C. Chromatography was performed at 4°C. Presence of 6xHis-Tag IN/CCD in collected fractions was assessed by electrophoresis on NuPAGE Bis-Tris 10% acrylamide gels with MES as electrophoresis buffer (Invitrogen). Proteins were stained using Imperial Protein Stain™ (Thermo Scientific Pierce). Pooled fractions from Superdex200 or Superdex75 separation were concentrated and stored at -80°C until further used.
- GST-tagged Flag-CCD or GST-tagged Flag-IBD lysates were loaded at 0.25 mL/min on a 20 mL Glutathione Sepharose 4 fast-flow (GE Healthcare) column. Bound proteins were eluted using integrase CCD buffer with 20 mM reduced glutathione added to it. Purification was completed as described above.
- Flag-IN was prepared from a GST-Flag-IN fusion protein expressed using the pGEX-6P expression system (GE Healthcare). After adsorption to the Glutathione Sepharose 4 fast-flow column, protein corresponding to 1 liter culture extract was digested while in the column by 250 units Prescission protease (GE Healthcare) 16 hours at 4°C. Cleaved protein was eluted by restarting the buffer flow over the column. Purification was completed by gel filtration on Superdex 200 as described above.
- rGST was purified on Glutathione Sepharose 4 fast-flow and Superdex 75 16/600 PG columns as described above except that PBS buffer was used.

**Size Exclusion Chromatography:** was performed on a Superdex 200 PC 3.2/30 column (GE Healthcare) using an AKTA chromatography system (GE Healthcare) at 0.04 mL/min at room temperature. 20 µL of sample was injected on the column previously equilibrated in Integrase buffer (50 mM HEPES pH 7.5 containing 1 M NaCl, 7 mM CHAPS, 5 mM MgCl2, 10 % glycerol). 1 µM Compound was added to the mobile phase in order to avoid integrase-inhibitor dissociation during the separation. His-Integrase (8 µM) was incubated 10 min at room temperature in the presence of 20 µM compound prior to injection on the column (hence a ratio Cpd-IN of 2.5).

### Exemple 5: Virus inactivation assays for vaccine preparation

### I- Reagents and Assays for the identification of antiretroviral compounds capable to inactivate HIV by treatment of 293T producer cells transfected by plasmids harboring HIV infectious molecular clones:

### Reagents:

293T cell line
MT4 cell line
Plasmid harboring HIV full length infectious molecular clone such as pNL4-3
Transfection reagent such as X-tremeGENE 9 reagent (Roche)
RPMI medium
PBS buffer
CellTiter-Glo®luminescent reagent (Promega)
Alliance HIV-1 p24 Antigen ELISA (PerkinElmer, http://www.perkinelmer.com/) 96-well white plates (Costar)
DMSO
Protease inhibitor
This assay kit comprises several steps that are detailed below
**1- Antiviral assays to characterize compounds with antiretroviral activity:**
   **a) replication-competent HIV assay (Multiple round infection assay) using MT-4 cells as target cells:** MT-4 cells growing exponentially at the density of 10⁶/ml are infected with an HIV-1 strain such as NL4-3 or HXB2 during two hours. The cells are then washed with PBS and then aliquoted in 100 µL fresh complete RPMI to 96-well white plates (Costar) in the presence of varying concentrations of compounds. The effective concentration of compound required to inhibit 50% (EC₅₀) of HIV-1 replication is determined after 5 days using the CellTiter-Glo^{®} luminescent reagent (Promega) to quantify cell viability.
   **b) Replication-defective-HIV assay:** MT-4 cells growing exponentially at the density of 10⁶/ml are infected with VSV pseudotyped NL4-3Δ*env*-luc during 90 minutes. The cells are then washed with PBS and then aliquoted in 100µl fresh complete RPMI to 96-well white plates (Costar) in the presence of varying concentrations of compounds. Luciferase expression as a control of HIV infection is read two days later using the One-Glo™ luciferase assay (Promega). The effective concentration of compound is the concentration required to inhibit 50% (EC₅₀) of HIV-1 replication.
**2- Production of Viral particles in the presence of compounds.** 293T cells (2.2 10⁶ cells) are transfected with plasmids harboring full length cloned HIV proviral DNA such as pNL4-3 or any other HIV proviral clone including autologous HIV molecular clones using DNA transfection reagent such as X-tremeGENE 9 reagent (Roche). Cells are washed 3h later, trypsinized and diluted at 0.3 10⁶ cells per ml. 5 10⁵ cells in 1.6ml fresh culture media are distributed into 6 wells plate and the volume is adjusted to 2ml by adding 0.4 ml of media containing compounds and DMSO per well, or DMSO only as control. As reference compounds capable to fully inactivate HIV during virus production by producer cells, Protease inhibitors such as Indinavir or Saquinavir are used as additional controls. Final concentration for each compound, including reference protease inhibitor compounds, is kept equivalent to 5 times its EC50 concentration previously calculated into a multiple round assay as in (a) and DMSO is kept at 0.5% final concentration. Supernatants containing HIV virions are collected 48 h post-transfection and stored at - 80°C. All Viral stocks are quantified for p24 antigen using the Alliance HIV-1 p24 Antigen ELISA (PerkinElmer, http://www.perkinelmer.com/) and titrated to measure the quantity of infectious particles per ml by infecting TZM-bl indicator cells.
**3- Infection with Virus Stock from treated producer cells to demonstrate virus inactivation by compounds:** Infection either of MT4 cells used as target cells are performed as described in 1a and 1b above, with serial dilution of the virus stock to ensure that incoming compounds did not interfere with the infection procedure. Usually 1/2000 dilution of the virus stock is used to infect MT4 target cells. Productive HIV-1 infection is determined after 5 days using the CellTiter-Glo^{®} luminescent reagent (Promega, France) to quantify MT4 cell viability. Alternatively, productive infection can be estimated by quantitation of p24 antigen as described in paragraph 2 above. Full inactivation of the virus stock by compounds is estimated by results obtained in the presence of compound compared on the one hand to DMSO alone which indicates the 100% infectious virus stock (0% inactivation), and on the other hand to Protease inhibitor treatment which indicates the 100% virus stock inactivation.

### II- Reagents and Assays for the identification of antiretroviral compounds capable to inactivate HIV by treatment of Hela-LAV producer cell line:

Hela-LAV cell line
MT4 cell line
RPMI medium
PBS buffer
CellTiter-Glo^{®} luminescent reagent (Promega)
Alliance HIV-1 p24 Antigen ELISA (PerkinElmer, http://www.perkinelmer.com/) 96-well white plates (Costar)
DMSO
Protease inhibitor
This assay kit comprises several steps that are detailed below
**1- Antiviral assays to characterize compounds with antiretroviral activity:**
   **a) replication-competent HIV assay (Multiple round infection assay) using MT-4 cells as target cells:** MT-4 cells growing exponentially at the density of 10⁶/ml are infected with an HIV-1 strain such as NL4-3 or HXB2 during two hours. The cells are then washed with PBS and then aliquoted in 100 µL fresh complete RPMI to 96-well white plates (Costar) in the presence of varying concentrations of compounds. The effective concentration of compound required to inhibit 50% (EC₅₀) of HIV-1 replication is determined after 5 days using the CellTiter-Glo^{®} luminescent reagent (Promega) to quantify cell viability.
   **b) Replication-defective-HIV assay:** MT-4 cells growing exponentially at the density of 10⁶/ml are infected with VSV pseudotyped NL4-3Δ*env*-luc during 90 minutes. The cells are then washed with PBS and then aliquoted in 100µl fresh complete RPMI to 96-well white plates (Costar) in the presence of varying concentrations of compounds. Luciferase expression as a control of HIV infection is read two days later using the One-Glo™ luciferase assay (Promega). The effective concentration of compound is the concentration required to inhibit 50% (EC₅₀) of HIV-1 replication.
**2- Production of Viral particles in the presence of compounds.** Hela-LAV cells are treated with inactivating antiretroviral compound (Mut145509) or reference antiretroviral drugs like Raltegravir that are not active at production stage, or Protease inhibitors such as Saquinavir (SQV) that are able to inactivate HIV at production stage or DMSO as negative control. Final concentration for each compound, including reference protease inhibitor compounds, is kept equivalent to 5 times its EC50 concentration previously calculated into a multiple round assay as in (a) and DMSO is kept at 0.5% final concentration. In order to examine the infectivity of viruses produced in the presence of these various compounds, the supernatants are harvested, stored at -80°C and titrated for viral protein p24 release using the Alliance HIV-1 p24 Antigen ELISA (PerkinElmer, http://www.perkinelmer.com/) and titrated to measure the quantity of infectious particles per ml by infecting TZM-bl indicator cells expressing luciferase under a Tat-dependent promoter. Alternatively for titration of infectious particles, target cells for HIV-1 infection such as MT4 cells are used as described above in 1a.
**3- Infection with Virus Stock from treated producer cells to demonstrate virus inactivation by compounds:** Infection either of MT4 cells used as target cells are performed as described in 1a and 1b above, with serial dilution of the virus stock to ensure that added compounds during virus production did not interfere with the infection procedure. Usually 1/2000 dilution of the virus stock is used to infect MT4 target cells. Productive HIV-1 infection is determined after 5 days using the CellTiter-Glo^{®} luminescent reagent (Promega, France) to quantify MT4 cell viability. Alternatively, productive infection can be estimated by quantitation of p24 antigen as described in paragraph 2 above. Full inactivation of the virus stock by compounds is estimated by results obtained in the presence of compound compared on the one hand to DMSO alone which indicates the 100% infectious virus stock (0% inactivation), and on the other hand to Protease inhibitor treatment which indicates the 100% virus stock inactivation.

### III- Reagents and Assays for the identification of antiretroviral compounds capable to inactivate autologous HIV primary isolates from HIV-infected patients produced by activated-PBMCs from infected patients co-cultured with PBMCs from HIV-negative individuals:

All reagents used to inactivate autologous HIV isolates from patients are sterile, endotoxin free and manufactured under GMP conditions. CD4-enriched PBMCs depleted from CD+ lymphocites using microBeads according the manufacturer's instructions, are obtained from blood buffy coats of HIV-negative donors and from HIV-infected patients after ficoll centrifugation. PBMCs obtained by ficoll centrifugation. PBMCs are stimulated with anti-CD3 (10ng/ml) and IL2 (10U:m1). For each HIV-1 infected subject, the primary autologous HIV-1 are prepared by co-culture of CD4-enriched PBMCs from infected patients with pre-activated CD4-enriched PBMCs from a healthy donor in the presence of IL2 at 10U/ml. Simultaneously, the autologous virus produced by such co-culture is inactivated by treatment of the co-culture with effective concentration of inactivating compound. Half of the volume of the cell co-culture supernatant is replaced with fresh medium after several days and the cell culture is fed by pre-activated CD4-enriched PBMCs from healthy donor, still in the presence of effective concentration of inactivating compound. The co-culture procedure can be repeated. Several days later, the entire co-culture supernatant is recovered by centrifugation for isolation of the inactivated-autologous virus. Inactivated HIV is then analyzed by testing supernatants for both HIV-1 p24 antigen-ELISA (Ag HIV Innogenetics K1048) and for absence of infectivity by infection of MT4 cells.

### IV- Identification of inactivating compounds by inhibition of IN-LEDGF interaction using HTRF® method (Homogeneous Time Resolved Fluorescence)

IN-LEDGF HTRF® assay was performed in 384-well low volume black polystyrene plates (Corning #3677) in IN-LEDGF assay buffer (25 mM Tris-HCl pH 7.4, 150 mM NaCl, 2 mM MgCl₂, 0.4 M KF, 0.1% Igepal CA-630, 0.1% bovine serum albumin, 1 mM DTT). 2 µL of 3-fold serial dilutions of inhibitory compound in 25% DMSO were preincubated for 30 min at room temperature with 8 µL of IN mixture (50 nM Flag-tagged IN, 17 nM XL₆₆₅-conjugated anti-Flag M2 monoclonal antibody (Cisbio Bioassays #61FG2XLB)). Then, 10 µL of LEDGF mixture (60 nM His₆-tagged LEDGF/p75, 1.5 nM Terbium cryptate-labeled anti-His₆ monoclonal antibody (Cisbio Bioassays #61HISTLB)) were added and the plate was incubated for 2.5h at room temperature before reading the time-resolved fluorescence in a BMG Labtech PHERAstar Plus (BMG Labtech) with HTRF module (excitation at 337 nm, dual emission at 620 nm and 667 nm). The HTRF ratio was converted to % inhibition and analyzed by fitting with a sigmoidal dose-response equation with Hill slope to determine the compound IC₅₀-

### V- Identification of inactivating compounds by enhancement of IN-IN multimerization interaction using HTRF® method (Homogeneous Time Resolved Fluorescence)

IN-IN HTRF® assay was performed in 384-well low volume black polystyrene plates (Corning #3677). 2 µL of 3-fold serial dilutions of inhibitory compound in 25% DMSO were preincubated for 30 min at room temperature with 4 µL of 125 nM Flag-IN dilution. Then, 4 µL of 125 nM 6xHis-IN were added and the plate was incubated 3h at room temperature to allow IN subunit exchange and multimerization. This step was performed in IN2 buffer (25 mM HEPES pH 7.4, 150 mM NaCl, 2 mM MgCl₂, 0.005% Tween-20, 0.1% bovine serum albumin, 1 mM DTT). Finally, 10 µL of revelation mixture (1.1 nM Europium cryptate-labeled monoclonal anti-Flag M2 antibody (Cisbio Bioassays #61FG2KLB), 13 nM XL₆₆₅-labeled anti-His₆ monoclonal antibody (Cisbio Bioassays #61HISXLB) in IN2 buffer supplemented with 0.8 M KF) were added and the plate was incubated for 2 more hours at room temperature before reading the time-resolved fluorescence in a BMG Labtech PHERAstar Plus (BMG Labtech) with HTRF module (excitation at 337 nm, dual emission at 620 nm and 667 nm). The HTRF ratio was converted to % activation and analyzed by fitting with a sigmoidal dose-response equation with Hill slope to determine the compound AC₅₀ and activation plateau.

## Claims

**1.** A method for producing an immunogenic composition or vaccine comprising inactivated lentivirus, in particular inactivated HIV, wherein producer cells constituvely producing lentivirus particles are provided, the lentivirus particles are produced by these producer cells in the presence of an antiretroviral (ARV) agent which is an inhibitor of the IN-LEDGF/p75 interaction, the inactivated lentivirus is recovered and formulated in a pharmaceutically acceptable vehicle or carrier.

**2.** The method of claim 1, wherein the inactivated lentivirus is recovered and formulated in a pharmaceutically acceptable vehicle or carrier and an adjuvant.

**3.** The method of claim 1 or 2, wherein the inactivated lentivirus is recovered and formulated in a pharmaceutically acceptable vehicle or carrier, optionally an adjuvant, and the formulation is sterilized.

**4.** The method of any one of the preceding claims, wherein the producer cell is a cell line which expresses constituvely lentivirus particles.

**5.** The method of any one of the preceding claims, wherein the producer cells are transfected with a plasmid harboring full length lentiviral proviral DNA construct.

**7.** The method of any one of the preceding claims, wherein the producer cell harbour CD4 receptor and/or the co-receptor CCR5 and/or CXCR4.

**8.** The method of any one of the preceding claims, wherein the inactivated lentivirus comprises a multimerized form of inactive integrase having a molecular weight greater than the integrase dimer.

**9.** The method of any one of the preceding claims, wherein the inactivated lentivirus comprises an inactive tetramer of integrase.

**10.** The method of any one of the preceding claims, wherein the inactivated lentivirus comprises an inactive integrase having shifted toward higher order oligomerization such as an an inactive integrase tetramer of about 130 KD MW as estimated using the method of size exclusion chromatography on a Superdex PC 3.2/30 column (GE Healthcare).

**11.** The method of any one of the preceding claims, comprising the binding of the ARV agent to the LEDGF-binding pocket on lentivirus integrase, especially HIV integrase.

**12.** The method of any one of the preceding claims, wherein the inactivated lentivirus is an inactivated VLP.

**13.** The method of any one of the preceding claims, wherein the ARV agent is a compound of formula (1) or (2) : 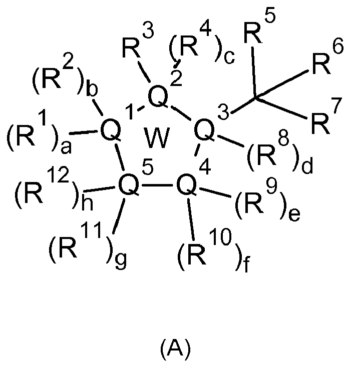 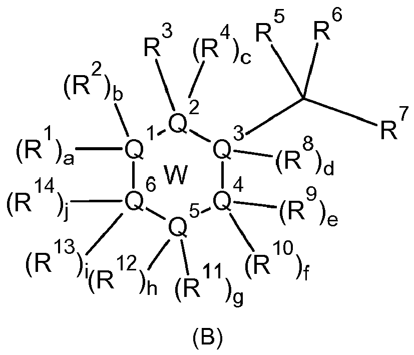 wherein :
• W represents a substituted or non-substituted, partially or totally unsaturated, aromatic or non-aromatic carbo- or heterocycle ;
• a, b, c, d, e, f, g, h, i and j independently represent 0 or 1;
• Q¹ represents CR¹, CR², CR¹R², N, NR¹NR²,S, O, C=O, C=S, S=O, S(O)₂;
• Q² represents CR³,CR⁴, CR³R⁴, NR³, NR⁴;
• Q³ represents CR⁸(CR⁵R⁶R⁷), CR⁸(R⁸CR⁵R⁶R⁷), N(CR⁵R⁶R⁷);*
• Q⁴ represents CR⁹, CR¹⁰, CR⁹R¹⁰, N, NR⁹, NR¹⁰, S, O, C=O, C=S, S=O, S(O)₂;
• Q⁵ represents CR¹¹, CR¹², CR¹¹R¹², N, NR¹¹, NR¹², S, O, C=O, C=S, S=O, S(O)₂;
• Q⁶ represents CR¹³, CR¹⁴, CR¹³R¹⁴ N, NR¹³, NR¹⁴, S, O, C=O, C=S, S=O, S(O)₂;
• R¹, R², R⁹, R¹⁰, R¹¹, R¹², R¹³ and R¹⁴, non-substituted or substituted by at least one T¹, independently represent a hydrogen atom, -CN, -OH, -O-cycloalkyl, -O-cycloalkenyl, -O-cycloalkynyl, -NH₂, -NR¹⁵-cycloalkyl, -NR¹⁵-cycloalkenyl, -NR¹⁵-cycloalkynyl, -S-cycloalkyl, -S-cycloalkenyl, -S-cycloalkynyl, -COOR¹⁵, -OC(O)R¹⁶ , -C(O)NR¹⁵R¹⁶, -NR¹⁶C(O)R¹⁵, -CF₃, -SO₃R¹⁵, -SO₂NR¹⁵R¹⁶, -NR¹⁶SO₂R¹⁵-NR¹⁶SO₂NR¹⁵R¹⁶,- NR¹⁶C(O)NR¹⁵R¹⁶, -OC(O)NR¹⁵R¹⁶,- NR¹⁶C(O)O, halogen, alkyl, alkenyl, alkynyl, heteroalkyl, heteroalkenyl, heteroalkynyl, fluoroalkyl, fluoroalkenyl, fluoroalkynyl, aryl, -O-aryl, -NR¹⁵-aryl, -S-aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, -O-heterocycle, -NR¹⁵-heterocycle, -S-heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl; wherein a carbon atom or a heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl can be oxidized to form a C=O, C=S, S=O, S(O)₂ or S(O)₃H;
wherein the aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, or heterocyclyl-heteroalkynyl group can be fused with at least one further cycle ;
and wherein the alkyl, alkenyl, alkynyl, arylalkyl, arylalkenyl, arylalkynyl, cycloalkyl, cycloalkenyl, cycloalkynyl, heterocyclyl-alkyl, heterocyclyl-alkenyl, heterocyclyl-alkynyl group can include one or more heteroatoms, selected from O, S and N, within the alkyl, alkenyl, alkynyl moiety ;
R³, non-substituted or substituted by at least one T¹, represents -O-cycloalkyl, -O-cycloalkenyl, -O-cycloalkynyl, -NR¹⁵-cycloalkyl, -NR¹⁵-cycloalkenyl, -NR¹⁵-cycloalkynyl, -S-cycloalkyl, -S-cycloalkenyl, -S-cycloalkynyl, alkyl comprising at least 4 carbon atoms, alkenyl comprising at least 4 carbon atoms, alkynyl comprising at least 4 carbon atoms, heteroalkyl, heteroalkenyl, heteroalkynyl, aryl, -O-aryl, -NR¹⁵-aryl, -S-aryl,arylalkyl, arylalkenyl, arylalkynyl, arylheteroalkyl, arylheteroalkenyl, arylheteroalkynyl, cycloalkyl, cycloalkenyl, cycloalkynyl,
heterocycle, -O-heterocycle, -NR¹⁵-heterocycle, -S-heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl; wherein a carbon atom or heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, heterocycle, heterocyclyl-alkyl, heterocylyl-alkenyl, heterocyclyl-alkynyl, heterocyclyl-heteroalkyl, heterocylyl-heteroalkenyl, heterocyclyl-heteroalkynyl can be oxidized to form a C=O, C=S, S=O, S(O)₂ or S(O)₃H;
wherein the aryl, arylalkyl, arylalkenyl, arylalkynyl, arylheteroalkyl, arylheteroalkenyl, arylheteroalkynyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, or heterocyclyl-heteroalkynyl can be fused with at least one further cycle;
and wherein alkyl, alkenyl, alkynyl, arylalkyl, arylalkenyl, arylalkynyl, cycloalkyl, cycloalkenyl, cycloalkynyl, heterocyclyl-alkyl, heterocyclyl-alkenyl, heterocyclyl-alkynyl group can include one or more heteroatoms, selected from O, S and N, in the alkyl, alkenyl, alkynyl moiety ;
• R⁴ represents a hydrogen atom, -OH, alkyl, alkenyl, alkynyl, O-alkyl, O-alkenyl, O-alkynyl, aryl, cycloalkyl, heterocycle;
wherein alkyl, alkenyl, alkynyl or heterocycle group can include one or more heteroatoms, selected from O, S and N ;
wherein a carbon atom or heteroatom of said alkyl, alkenyl, alkynyl or heterocycle group, can be oxidized to form a C=O, C=S, S=O, S(O)₂ or S(O)₃H;
• R⁵ or R⁶, non-substituted or substituted by at least one T², identical, or different, independently represent a hydrogen atom, halogen, -CN, -O-cycloalkyl, -O-cycloalkenyl, -O-cycloalkynyl, -NR¹⁵-cycloalkyl, -NR¹⁵-cycloalkenyl, -NR¹⁵-cycloalkynyl, -S-cycloalkyl, -S-cycloalkenyl, -S-cycloalkynyl, alkyl, alkenyl, alkynyl, heteroalkyl, heteroalkenyl, heteroalkynyl, aryl, -O-aryl, -NR¹⁵-aryl, -S-aryl,arylalkyl, arylalkenyl, arylalkynyl, arylheteroalkyl, arylheteroalkenyl, arylheteroalkynyl, cycloalkyl, cycloalkenyl, cycloalkynyl, heterocycle, -O-heterocycle, -NR¹⁵-heterocycle, -S-heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl;
wherein a carbon atom or heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynylcan be oxidized to form a C=O, C=S, S=O, S(O)₂ or S(O)₃H;
wherein alkyl, alkenyl, alkynyl, arylalkyl, arylalkenyl, arylalkynyl, cycloalkyl, cycloalkenyl, cycloalkynyl, heterocyclyl-alkyl, heterocyclyl-alkenyl, heterocyclyl-alkynyl group can include one or more heteroatoms, selected from O, S and N, in the alkyl, alkenyl, alkynyl moiety;
R⁵ and R⁶ form with the carbon atom to which they are bonded, a 3- to 7-membered carbocycle or heterocycle,
wherein the carbocycle or heterocycle is fused with at least one further cycle, or R⁵ and R⁶ form a group of formula (i) 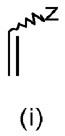 wherein Z represents a hydrogen atom, alkyl or heteroalkyl and wherein a carbon atom or heteroatom of said alkyl, can be oxidized to form a C=O, C=S, S=O, S(O)₂ or S(O)₃H;
• R⁷ represents independently -C(O)OH, -CN, -C(O)NH₂, -C(O)OR¹⁵, -C(O)NHCN, - C(O)NHOH, -S(O)₂OH, -S(O)₂NHR¹⁵, -P(O)(OH)NH₂, -P(O)(OH)O-alkyl, -P(O)(O-alkyl)₂, -P(O)(OH)₂, -OSO₃H, -NR¹⁵SO₃H, a tetrazolyl group;
• R⁸ represents a hydrogen atom, alkyl, alkenyl, alkynyl;
• R¹⁵, identical or different, independently represents a hydrogen atom, alkyl, alkenyl, alkynyl, haloalkyl, haloalkenyl, haloalkynyl, cycloalkyl,cycloalkenyl,aryl, heterocycle, alkylcycloalkyl, alkylcycloalkenyl, alkenylcycloalkyl, alkynylcycloalkyl, alkenylcycloalkenyl, heterocycle, alkylheterocycle, alkenylheterocycle, alkynylheterocycle
wherein a carbon atom of said alkyl or aryl can be oxidized to form a C=O, C=S;
• R¹⁶, identical or different, independently represents a hydrogen atom, alkyl, alkenyl, alkynyl, haloalkyl, haloalkenyl, haloalkynyl, cycloalkyl,cycloalkenyl,aryl, heterocycle, alkylcycloalkyl, alkylcycloalkenyl, alkenylcycloalkyl, alkynylcycloalkyl, alkenylcycloalkenyl, heterocycle, alkylheterocycle, alkenylheterocycle, alkynylheterocycle,;
wherein a carbon atom of said alkyl or aryl can be oxidized to form a C=O, C=S;
• R¹⁵ and R¹⁶ form, with the azote atom to which they are bonded, a heterocycle comprising at least one N atom;
• T¹, identical or different, independently represents a hydrogen atom, halogen,-OT³, -OCF₃, =O, -ST³, =S, -S(O)T⁴, -S(O)₂T⁴ -S(O)₂NT⁵T⁶, CF₃, NO₂, -NT⁵T⁶,-NT³S(O)₂T⁴, CN, -NT³C(O)T⁴, -NT³C(O)NT⁵T⁶, -C(O)OT³, -C(O)NT⁵T⁶, -C(O)T⁴, alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl,heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl:
wherein said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl,heterocycle, heterocyclyl-alkyl, heterocylyl-alkenyl, heterocyclyl-alkynyl, heterocyclyl-heteroalkyl, heterocylyl-heteroalkenyl, heterocyclyl-heteroalkynyl can be substituted with one or more T⁷;
wherein a carbon atom or heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl,heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl can be oxidized to form a C=O, C=S, S=O, S(O)₂ or S(O)₃H;
• T², identical or different, independently represents a hydrogen atom, halogen,-OT⁸, -OCF₃, =O, -ST⁸, =S, -S(O)T⁹, -S(O)₂T⁹, -S(O)₂NT¹⁰T¹¹, -CF₃, -NO₂, -NT¹⁰T¹¹, -NT⁸S(O)₂T⁹, -CN, -NT⁸C(O)T⁹, -NT⁸C(0)NT¹⁰T¹¹, -C(O)OT⁸, -C(O)NT¹⁰T¹¹,-C(O)T⁹, alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl,heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl;
wherein said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkenyl, cycloalkynyl,heterocycle, heterocyclyl-alkyl, heterocylyl-alkenyl, heterocyclyl-alkynyl, heterocyclyl-heteroalkyl, heterocylyl-heteroalkenyl, heterocyclyl-heteroalkynyl can be substituted with one or more T⁷; wherein a carbon atom or heteroatom of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, cycloalkyl, cycloalkyl, cycloalkenyl, cycloalkynyl, heterocycle, heterocyclyl-alkyl, heterocyclyl-alkenyl, heterocyclyl-alkynyl, heterocyclyl-heteroalkyl, heterocyclyl-heteroalkenyl, heterocyclyl-heteroalkynyl can be oxidized to form a C=O, C=S, S=O, S(O)₂ or S(O)₃H;
• T³, identical or different, independently represents a hydrogen atom, alkyl, alkenyl, alkynyl, heteroalkyl, heteroalkenyl, heteroalkynyl, aryl, heterocycle;
wherein said alkyl, alkenyl, alkynyl, heteroalkyl, heteroalkenyl, heteroalkynyl, aryl, heterocycle can be substituted or non substituted with one or more -OH, =O, halogen, -SH, =S, -CF₃, -O-alkyl, -OCF₃, -CN, -NO₂, -C(O)OH, -NH₂ or C(O)NH₂; wherein a carbon atom or heteroatom of said alkyl, alkenyl, alkynyl, heteroalkyl, heteroalkenyl, heteroalkynyl, aryl, heterocycle can be oxidized to form a C=O, C=S, S=O, S(O)₂ or S(O)₃H;
• T⁴, identical or different, independently represents -OH, alkyl, alkenyl, alkynyl, heteroalkyl, heteroalkenyl, heteroalkynyl, aryl, heterocycle;
wherein said alkyl, alkenyl, alkynyl, heteroalkyl, heteroalkenyl, heteroalkynyl, aryl, heterocycle, can be substituted or non substituted with one or more -OH, =O, halogen, -SH, =S, -CF₃, -O-alkyl, -OCF₃, -CN, -NO₂, -C(O)OH, -NH₂ or C(O)NH₂; wherein a carbon atom or heteroatom of said alkyl, alkenyl, alkynyl, heteroalkyl, heteroalkenyl, heteroalkynyl aryl, heterocycle can be oxidized to form a C=O, C=S, S=O,S(O)₂ or S(O)₃H;
• T⁵ or T⁶, identical or different, independently represent a hydrogen atom, alkyl, alkenyl, alkynyl, heteroalkyl, heteroalkenyl, heteroalkynyl, aryl, heterocycle wherein said alkyl, alkenyl, alkynyl, heteroalkyl, heteroalkenyl, heteroalkynyl, aryl, heterocycle can be substituted or non substituted with one or more -OH, =O, halogen, -SH, =S, -CF₃, -O-alkyl, -OCF₃, -CN, -NO₂, -C(O)OH, -NH₂ or C(O)NH₂; wherein a carbon atom or heteroatom of said alkyl, alkenyl, alkynyl, heteroalkyl, heteroalkenyl, heteroalkynyl aryl, heterocycle can be oxidized to form a C=O, C=S, , S=O, S(O)₂ or S(O)₃H;
or T⁵ or T⁶ can be form, with the azote atom to which they are bonded, a 4-, 5-, 6-or 7- membered heterocycle non substituted or substituted with an alkyl, alkenyl, alkynyl, heteroalkyl, heteroalkenyl, heteroalkynyl, -OH, halogen, -SH, -CF₃, -O-alkyl, -OCF₃, -CN, -NO₂, -C(O)OH, -NH₂ or -C(O)NH₂;
• T⁷, identical or different, independently represents an alkyl, alkenyl, alkynyl, heteroalkyl, heteroalkenyl, heteroalkynyl, -OH, =O, halogen, -SH, =S, -CF₃, -CN, - NO₂, -C(O)OH, -NH₂ or-C(O)NH₂;
• T⁸, identical or different, independently represents a hydrogen atom, alkyl, alkenyl, alkynyl, heteroalkyl, heteroalkenyl, heteroalkynyl;
wherein said alkyl, alkenyl, alkynyl, heteroalkyl, heteroalkenyl, heteroalkynyl, can be substituted or non substituted with one or more -OH, =O, halogen, -SH, =S,-CF₃, -O-alkyl, -OCF₃, -CN, -NO₂, -C(O)OH, -NH₂ or -C(O)NH₂;
wherein a carbon atom or heteroatom of said alkyl, alkenyl, alkynyl, heteroalkyl, heteroalkenyl, heteroalkynyl can be oxidized to form a C=O, C=S, S=O, S(O)₂ or S(O)₃H;
• T⁹, identical or different, independently represents -OH, alkyl, alkenyl, alkynyl, heteroalkyl, heteroalkenyl, heteroalkynyl, wherein said alkyl, alkenyl, alkynyl, heteroalkyl, heteroalkenyl, heteroalkynyl, can be substituted or non substituted with one or more -OH, =O, halogen, -SH, =S, -CF₃, -O-alkyl, -OCF₃, -CN, -NO₂, - C(O)OH, -NH₂ or -C(O)NH₂;
wherein a carbon atom or heteroatom of said alkyl, alkenyl, alkynyl, heteroalkyl, heteroalkenyl, heteroalkynyl can be oxidized to form a C=O, C=S, S=O, S(O)₂ or S(O)₃H;
• T¹⁰ or T¹¹, identical or different, independently represent a hydrogen atom, alkyl, alkenyl, alkynyl, heteroalkyl, heteroalkenyl, heteroalkynyl;
wherein said alkyl, alkenyl, alkynyl, heteroalkyl, heteroalkenyl, heteroalkynyl, can be substituted or non substituted with one or more -OH, =O, halogen, -SH, =S,-CF₃, -O-alkyl, -OCF₃, -CN, -NO₂, -C(O)OH, -NH₂ or -C(O)NH₂;
wherein a carbon atom or heteroatom of said alkyl, alkenyl, alkynyl, heteroalkyl, heteroalkenyl, heteroalkynyl can be oxidized to form a C=O, C=S, S=O, S(O)₂ or S(O)₃H;
or T¹⁰ or T¹¹ can be form, with the azote atom to which they are bonded, a 4-, 5-, 6-or 7- membered heterocycle non substituted or substituted with an alkyl, alkenyl, alkynyl, heteroalkyl, heteroalkenyl, heteroalkynyl, -OH, halogen, -SH, -CF₃, O-alkyl, -OCF₃, -CN, -N02, -C(O)OH, -NH₂ or -C(O)NH₂;
and a racemate, enantiomer, isomer or diastereoisomer or a phamaceutically acceptable salt thereof.

**14.** The method of any one of the preceding claims, comprising the preparation of a plasmid harboring an infectious lentivirus molecular clone from a previously cloned virus from a biobank or isolated from a lentivirus infected individual, or the preparation of plasmids harboring infectious lentivirus molecular clones prepared from the quasi species population of lentivirus that infect a patient.

**15.** The method of any one of claims 1-13, wherein the producer cells are cotransfected with a plasmid harboring lentiviral proviral DNA construct that does not express the envelope gene either by stop codon mutation or deletion, together with a plasmid encoding an exogenous viral envelope such as that of the vesicular stomatitis virus protein GVSVG.

**16.** The method of any one of the preceding claims, comprising further dendritic cells and the dendritic cells stimulated by loading with the inactivated lentivirus or the inactivated VLP.

**17.** An immunogenic composition or vaccine comprising an inactivated lentivirus, especially inactivated HIV, in a pharmaceutically acceptable carrier or vehicle, and optionally an adjuvant, obtained using the method according to any one of the preceding claims.

**18.** An immunogenic composition or vaccine comprising an inactivated lentivirus, especially inactivated HIV, in a pharmaceutically acceptable carrier or vehicle, and optionally an adjuvant, wherein the lentivirus comprises an inactive tetramer of integrase or a multimerized form of inactive integrase having a molecular weight greater than the integrase dimer.

**19.** An immunogenic composition or vaccine comprising an inactivated lentivirus, especially inactivated HIV, in a pharmaceutically acceptable carrier or vehicle, and optionally an adjuvant, wherein the lentivirus comprises an inactive integrase having shifted toward higher order oligomerization such as an an inactive integrase tetramer of about 130 KD MW as estimated using the method of size exclusion chromatography on a Superdex PC 3.2/30 column (GE Healthcare)

**20.** A kit for prophylactic or therapeutic treatment of a mammal, especially a human, against a lentivirus, especially HIV, comprising an immunogenic composition or vaccine according to claim 17, 18 or 19 and a antiretroviral agent, preferably an ARV agent which is an inhibitor of the IN-LEDGF/p75 interaction, for a simultaneous, separate or sequential administration.
